# EUROPÄISCHE PATENTSCHRIFT

(11) **EP 2 849 649 B1**
(45) Veröffentlichungstag und Bekanntmachung des Hinweises auf die Patenterteilung: **11.01.2017**
(21) Anmeldenummer: 13722765.8
(22) Anmeldetag: 16.05.2013
(51) Int. Cl.: A61B 6/00

(54) **RÖNTGENDETEKTOR MIT MANIPULATIONSEINRICHTUNG ZUM BEWEGEN VON DETEKTORMODULEN AUF EINER KREISBAHN**
X-RAY DETECTOR WITH MANIPULATION UNIT FOR MOVEMENT OF DETECTOR MODULES ON A CIRCLE
DÉTECTEUR DE RAYON X AVEC UN DISPOSITIF POUR BOUGER LES MODULES DE DÉTECTION SUR UN CERCLE

(30) Priorität: 16.05.2012 DE 102012208305
(43) Veröffentlichungstag der Anmeldung: 25.03.2015
(73) Patentinhaber: Fraunhofer Gesellschaft zur Förderung der angewandten Forschung e.V., 80686 München (DE)
(72) Erfinder: HOFMANN, Thomas, 91091 Großenseebach (DE); HEBELE, Stefan, 90459 Nürnberg (DE); SCHLECHTER, Thomas, 91315 Höchstadt (DE)
(74) Vertreter: Hersina, Günter
(86) Internationale Anmeldenummer: PCT/EP2013/060122
(87) Internationale Veröffentlichungsnummer: WO 2013/171295

(56) Entgegenhaltungen:
- EP-A1- 2 105 762
- WO-A2-02/079802
- WO-A2-2008/135994
- WO-A2-2009/125309
- WO-A2-2011/149181
- DE-A1-102007 035 673
- US-A- 4 204 123
- US-A1- 2008 039 721
- US-B1- 6 242 743
- US-B1- 6 583 420

## Beschreibung

Ausführungsbeispiele der vorliegenden Erfindung beziehen sich auf einen Röntgendetektor und insbesondere auf einen Röntgendetektor mit einstellbarem Krümmungsradius.

In Röntgensystemen sind Röntgendetektoren, wie z.B. CCD-Sensoren mit einem Szintillator, verbaut, um ein Absorptionsprofil zu detektieren, welches aus einer Durchstrahlung eines Objekts mit einer Röntgenstrahlung resultiert und einen Rückschluss auf eine Dichteverteilung des Objekts zulässt.

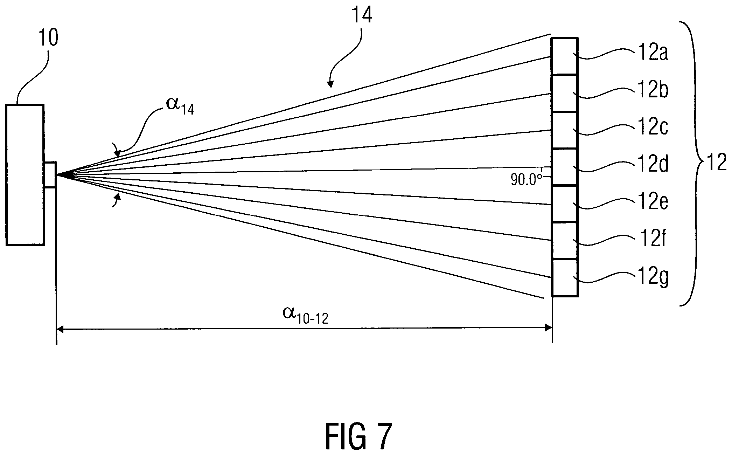

Fig. 7 zeigt ein derartiges Röntgensystem mit einer Strahlungsquelle 10, und einem Detektor 12, der in einem Fokus-Detektor-Abstand a_{10_12} von der Strahlungsquelle 10 beabstandet angeordnet ist. Der Detektor 12 weist sieben Detektormodule 12a bis 12g, z.B. sieben Detektorzeilen auf. Die Strahlungsquelle 10, wie z.B. eine Röntgenröhre, stellt eine punktförmige Strahlungsquelle mit einem Öffnungswinkel α₁₄ dar, so dass folglich die emittierte Röntgenstrahlung (vgl. Strahlengang 14) sich kugelförmig ausbreitet. Da der hier verwendete Detektor 12 flach ist, ist ein Auftreffwinkel der Röntgenstrahlung (vgl. Strahlengang 14) auf dem Detektor 12 von dem Detektorort (vgl. Detektormodul 12a bis 12g) abhängig. So trifft ein Zentralstrahl der Strahlungsquelle 10 senkrecht auf die Detektormitte bzw. auf das mittlere Detektormodul 12d auf, während die Röntgenstrahlung je flacher auf den Detektor 12 auftrifft, desto weiter das einzelne Detektormodul, z.B. 12a oder 12g, von der Mitte entfernt ist.

Flache Detektoren wie z.B. Flachbilddetektoren oder gerade Zeilendetektoren 12 zeichnen sich gegenüber gebogenen Detektoren durch ihren verhältnismäßig niedrigen Preis und eine einfache Handhabbarkeit, z.B. hinsichtlich Variierenbarkeit des Fokus-Detektor-Abstands a_{10_12}, aus, wobei allerdings die Bildqualität zu den Randbereichen hin abnehmen kann. Hintergrund hierzu ist, dass bei einem flacher werdenden Auftreffwinkel (z.B. beim Detektormodul 12a oder 12g) der Anteil der Röntgenstrahlung 14, der ein benachbartes Pixel kreuzt, zunimmt. Aufgrund der Schrägdurchstrahlung durch die Sensorschicht erfolgt eine Verschlechterung der Ortsauflösung bzw. genauer eine Signalverschmierung über die benachbarten Pixel hinweg. Folglich ist dieser Effekt am Rand (vgl. Detektormodul 12a bzw. 12g) maximal. Aus der gezeigten Konstellation zwischen einer punktförmigen Strahlungsquelle 10 und einem flächigen Detektor 12 entsteht ein weiterer Effekt, nämlich der, dass unterschiedliche Dosisraten aus den unterschiedlichen Abständen zwischen der Strahlungsquelle 10 und den Detektormodulen 12a bis 12g resultieren. So ist die Dosisrate auf die Detektormodule 12a und 12g im Randbereich im Vergleich zu der Dosisrate auf das Detektormodul 12d in der Mitte aufgrund des größeren Abstands geringer, was ein niedrigeres Signal und damit höheres Rauschen (niedriges Signal-Rausch-Verhältnis) zur Folge hat.

Es sei ferner angemerkt, dass bei gebogenen Detektoren (im Gegensatz zu Flachdetektoren), wie sie z.B. in Gantry-Systemen eingesetzt werden, der Auftreffwinkel ortsunabhängig ist bzw. sogar immer senkrecht ist, wenn der Fokus-Detektor-Abstand a_{10_12} (also Abstand des um das Messobjekt rotierenden Röhren-Detektor-Systems) nicht verändert wird. Insbesondere bei industriellen Anwendungen, bei denen sich die Messbedingungen (z.B. Aufstellung Strahlungsquelle 10 gegenüber Detektor 12) häufig ändern, werden allerdings gebogenen Detektoren aufgrund des nicht-veränderlichen Fokus-Detektor-Abstands a_{10_12} kaum eingesetzt.

Aufgabe der vorliegenden Erfindung ist es, einen flexibel einsetzbaren Röntgendetektor zu schaffen, der hinsichtlich seiner Bildqualität verbessert ist.

Die Aufgabe der vorliegenden Erfindung wird durch einen Röntgendetektor gemäß Anspruch 1 gelöst.

Ausführungsbeispiele der vorliegenden Erfindung schaffen einen Röntgendetektor mit einem ersten Detektormodul, einem zweiten Detektormodul und einer Manipulationseinrichtung. Die Detektormodule können beispielsweise einen oder mehrere Sensorelemente in ein oder mehreren Ebenen (zweidimensionaler Flächendetektor) aufweisen sowie die dazugehörige Elektronik und Mechanik umfassen. Das erste Detektormodul umfasst einen in einer ersten Erfassungsebene liegenden ersten Erfassungsbereich, das zweite Detektormodul umfasst einen in einer zweiten Erfassungsebene liegenden zweiten Erfassungsbereich, der benachbart oder angrenzend zu dem ersten Erfassungsbereich ist. Die Manipulationseinrichtung ist ausgebildet, um die erste Erfassungsebene des ersten Detektormoduls und die zweite Erfassungsebene des zweiten Detektormoduls so zueinander auszurichten, dass sich eine erste Flächennormale der ersten Erfassungsebene und eine zweite Flächennormale der zweiten Erfassungsebene innerhalb eines Referenzbereichs kreuzen.

Ausführungsbeispiele der vorliegenden Erfindung basieren darauf, dass Detektormodule kreisbogenförmig mit einem flexibel einstellbaren Krümmungsradius angeordnet werden. Durch die kreispunktförmige Anordnung der Detektormodule können sich alle Flächennormalen derselben in einem Referenzbereich, z.B. am Ort der Strahlungsquellen, treffen, was einer Fokussierung der Detektormodule auf die Strahlungsquelle gleichkommt. Somit fällt die Röntgenstrahlung immer senkrecht auf die einzelnen Detektormodule ein und eine Beeinflussung (Verschmierung) benachbarter Pixel infolge von Schrägdurchstrahlung kann minimiert werden. Der mittels einer Manipulationseinrichtung, z.B. einem Stellmotor, einstellbare Krümmungsradius ermöglicht eine Detektormodul-individuelle Ausrichtung des jeweiligen Erfassungsbereichs bzw. der jeweiligen Flächennormalen auf die Strahlungsquelle. Solch eine Vorrichtung ist bekannt aus US 6,583,420. Diese Einstellbarkeit schafft den Vorteil, dass eine exakte Fokussierung auch dann erreicht werden kann, wenn der Fokus-Detektor-Abstand, z.B. in Folge eines veränderten Messaufbaus, verändert wurde. Des Weiteren ist bei einer gebogenen Röntgendetektoranordnung die Strahlendosis der auf die einzelnen Detektormodule einfallenden Röntgenstrahlung unabhängig davon, auf welches Detektormodul die Röntgenstrahlung trifft, da der Abstand zwischen Strahlungsquelle und jedem Detektormodul aufgrund der kreisförmigen Anordnung immer gleich ist, wenn die Detektormodule auf die Strahlungsquelle bzw. auf einen Brennfleck der Strahlungsquelle ausgerichtet sind. Somit kann neben der Ortsauflösung gleichzeitig das Rauschverhalten über den gesamten Erfassungsbereich auf einen gleich guten Wert verbessert werden.

Entsprechend weiteren Ausführungsbeispielen sind die einzelnen Detektormodule flexibel, z.B. mit einer Feder oder einer anderen flexiblen mechanische Verbindung, relativ zueinander gelagert, so dass diese entlang des gewünschten Krümmungsradius angeordnet werden können. Zur Anordnung bzw. zur exakten Einstellung des Krümmungsradius kann die Manipulationseinrichtung ausgebildet sein, die zwei oder mehreren Detektormodule gleichmäßig entlang eines Kreisbogens auszurichten.

Ein weiteres Ausführungsbeispiel schafft ein Röntgendetektor mit einem ersten Detektormodul, einem zweiten Detektormodul, einem weiteren Detektormodul und einer Manipulationseinrichtung. Das erste Detektormodul weist einen ersten in einer ersten Erfassungsebene liegenden Erfassungsbereich auf, das zweite Detektormodul weist einen zweiten, in einer zweiten Erfassungsebene liegenden Erfassungsbereich auf und das weitere Detektormodul weist einen weiteren in einer weiteren Erfassungsebene liegenden weiteren Erfassungsbereich auf. Die erste und/oder zweite Erfassungsebene ist gegenüber der weiteren Erfassungsebene versetzt, wobei der erste und/oder zweite Erfassungsbereich mit dem weiteren Erfassungsbereich überlagert ist. Die Manipulationseinrichtung ist ausgebildet, um die erste Erfassungsebene des ersten Detektormoduls, die zweite Erfassungsebene des zweiten Detektormoduls und die weitere Erfassungsebene des weiteren Detektormoduls so zueinander auszurichten, dass sich eine erste Flächennormale der ersten Erfassungsebene, eine zweite Flächennormale der zweiten Erfassungsebene und eine weitere Flächennormale der weiteren Erfassungsebene innerhalb eines Referenzbereichs kreuzen. Hier sind somit vorteilhafterweise die einzelnen Detektormodule in zwei verschiedenen Reihen hintereinander angeordnet, so dass die gesamte Erfassungsebene durch die sich überlappende erste, zweite und weitere Erfassungsebene erfasst werden kann ohne dass sogenannte "tote Bereiche" an den Drehachsen zwischen zwei angrenzenden und hinsichtlich ihrer Flächennormalen einstellbaren Detektormodulen entstehen.

Ein weiteres Ausführungsbeispiel ist ein Röntgensystem mit einem oben beschriebenen Röntgendetektor und einer Strahlungsquelle, wobei der Referenzbereich in Abhängigkeit von einem Fokus-Detektor-Abstand mittels der Manipulationseinrichtung positionierbar ist.

Ausführungsbeispiele werden Bezug nehmend auf die beiliegenden Zeichnungen erläutert. Es zeigen:
- Fig. 1: eine schematische Prinzipdarstellung eines Röntgendetektors mit einstellbarem Krümmungsradius;
- Fig. 2a-b: eine schematische Prinzipdarstellung eines Röntgensystems ;
- Fig. 3a-c: eine schematische Prinzipdarstellung einer Manipulationseinrichtung gemäß einem Ausführungsbeispiel der Erfindung zusammen mit Diagrammen zur Illustration der auftretenden Kräfte und Momente;
- Fig. 4a: eine schematische Prinzipdarstellung eines Röntgendetektors mit zwei Krümmungsradien;
- Fig. 4b: eine schematische Prinzipdarstellung eines Röntgendetektors mit aufgebrachten Streustrahlrastern;
- Fig. 5a-5e: ein Konstruktionsbeispiel eines Röntgendetektors gemäß einem Ausführungsbeispiel mit Detailansichten und einem Flussdiagramm zur Illustration des Vorgangs des Einstellens der Detektormodule;
- Fig. 6a-6d: unterschiedliche Konstruktionsbeispiele für Manipulationseinrichtungen und
- Fig. 7: eine schematische Prinzipdarstellung eines Röntgendetektors gemäß dem Stand der Technik.

Bevor nachfolgend Ausführungsbeispiele anhand der Figuren näher erläutert werden, wird darauf hingewiesen, dass gleiche oder gleichwirkende Elemente mit gleichen Bezugszeichen versehen sind, so dass die Beschreibung derer aufeinander anwendbar bzw. austauschbar ist.

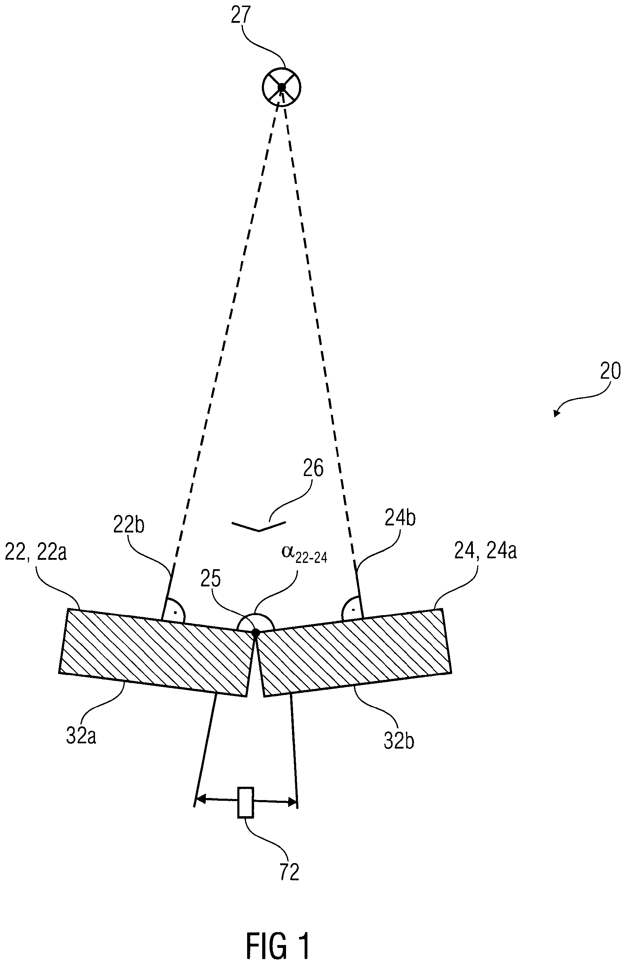

Fig. 1 zeigt einen Röntgendetektor 20 mit einem ersten Detektormodul 32a und einem zweiten Detektormodul 32b, mit denen eine Manipulationseinrichtung 72 zur exakten Positionierung derselben in Eingriff ist. Das erste Detektormodul 32a weist einen in einer ersten Erfassungsebene 22 liegenden ersten Erfassungsbereich 22a auf, das zweite Detektormodul 32b weist einen in einer zweiten Erfassungsebene 24 liegenden zweiten Erfassungsbereich 24a auf, wobei die zwei Erfassungsbereiche 22a und 24a so angeordnet sind, dass sie benachbart sind oder sogar direkt aneinander anstoßen und einer auftreffenden, von einer Strahlungsquelle 27 emittierten Röntgenstrahlung 26 zugewandt sind. Die zwei Detektormodule 32a und 32b sind relativ zueinander gewinkelt (vgl. Winkel α_{22_24}) bzw. auf einer Kreisbahn um die Strahlungsquelle 27 angeordnet, so dass jedes Detektormodul 32a und 32b direkt auf die Strahlungsquelle 27 ausgerichtet ist. In anderen Worten ausgedrückt, schneiden sich die Erfassungsebenen 22 und 24 in einer Achse 25, nämlich der Rotationsachse 25 der zwei Detektormodule 32a und 32b derart, dass eine erste Flächennormale 22b der ersten Erfassungsebene 22 und eine zweite Flächennormale 24b der zweiten Erfassungsebene 24 zur Strahlungsquelle 27 gerichtet sind bzw. sich innerhalb eines Referenzbereichs der Strahlenquelle 27 kreuzen.

Die Detektionsbedingungen sind umso besser, je kleiner der Referenzbereich ist, innerhalb welchem die Strahlungsquelle 27 (bzw. deren Brennfleck) positioniert ist. Somit sind die Detektionsbedingungen optimal, wenn sich die zwei Flächennormalen 22b und 24b am Ort der Strahlungsquelle 27 (Brennfleck) schneiden. Da der Fokus-Detektor-Abstand insbesondere bei industriellen Systemen nicht fix ist, sind die zwei Flächennormalen 22b und 24b bzw. die Anordnung der zwei Flächennormalen 22b und 24b zueinander justierbar. Hierbei kann die Fokussierung der zwei Detektormodule 32a und 32b auf die Strahlungsquelle 27 entsprechend einem gewählten Fokus-Detektor-Abstand (Entfernung zwischen dem Detektor 20 und der Strahlungsquelle 27) mittels der Manipulationseinrichtung 72 nachgestellt werden bzw. die Position des Referenzbereich bzw. Schnittpunkts der zwei Flächennormalen 22b und 24b so verändert werden, dass sich diese in der Nähe oder im Brennfleck der Strahlungsquelle 27 kreuzen. Hierzu ist beispielsweise der Winkel α_{22_24} zwischen den zwei Detektonnodulen 32a und 32b in Abhängigkeit von dem gewählten Fokus-Detektor-Abstand über die Manipulationseinrichtung 72 veränderbar, die zum Beispiel zwei Detektormodule 32a und 32b gegeneinander verdreht. Durch dieses Konzept ist es also möglich, einen senkrechten Einfallswinkel der Röntgenstrahlung 26 auf die Detektormodule 32a und 32b sicherzustellen, was zu einer verbesserten Abbildungsgenauigkeit (z.B. höheres Signal-Rausch-Verhältnis und/oder geringere Signalverschmierung) führt. Ferner wird bei Einsatz des Röntgendetektors 20 die Flexibilität des Röntgensystems erhöht, da mit demselben Messsystem sowohl kleine als auch große Objekte (d.h. unter Veränderung des Fokus-Detektor-Abstands) unter unterschiedlichen Ortsauflösungen untersucht werden können.

Entsprechend weiteren Ausführungsbeispielen sind die Detektormodule 32a und 32b so platziert, dass sie sich mit der Kante (vgl. Drehachse 25) der zur Röntgenstrahlung 26 hingewandten Seite (vgl. Erfassungsbereich 22a bzw. 24a) berühren oder nahezu berühren. Dies dient dazu, dass möglichst keine oder nur sehr kleine Lücken und damit kleine "Nicht-Erfassungsbereiche" oder "tote Bereiche" zwischen dem ersten und zweiten Erfassungsbereich 22a und 24a entstehen. Diese Berührkante ist auch gleichzeitig die Drehachse 25 (bzw. der Drehpunkt 25), um die sich die Detektormodule 32a und 32b bei einer Verstellung des Winkels α_{22_32b} relativ zueinander bewegen. Diese Drehachse 25 kann beispielsweise durch eine Lagerung der Detektormodule 32a und 32b definiert werden. Ein Beispiel hierfür wäre es, die Detektormodule 32a und 32b durch eine gemeinsame Feder, mittels welcher sie verbunden sind, relativ zueinander zu lagern, wobei die Drehachse 25 durch einen Knickpunkt der Feder definiert ist. Hierbei könnte mittels zusätzlicher Abstandshalter der Detektormodule 32a und 32b die Bewegungsgeometrie derselben genau festlegt werden.

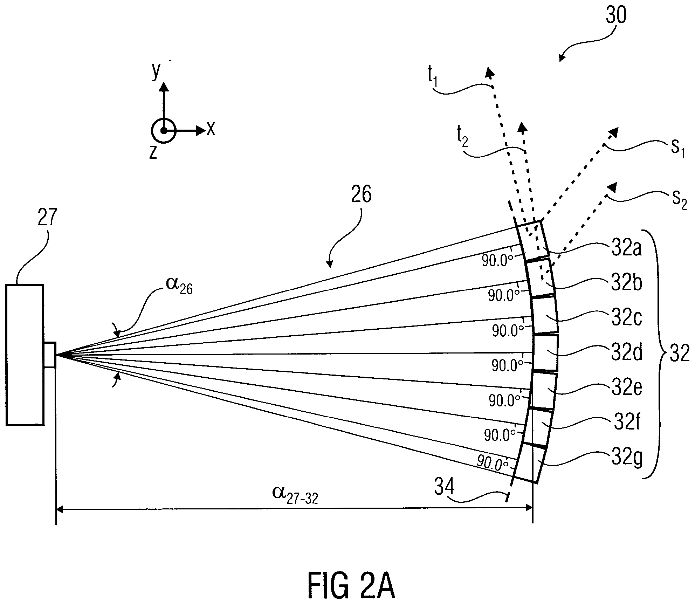

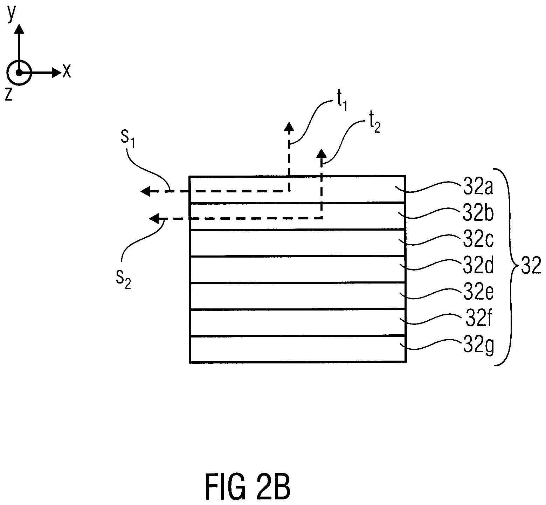

Fig. 2a zeigt ein Röntgensystem 30 mit einem Detektor 32 und die Strahlungsquelle 27, die die Röntgenstrahlung 26 kugelförmig emittiert. Hierbei wird in Fig. 2a das Röntgensystemen 30 in der xy-Ebene als Zeichenebene darstellt, während der Detektor 32 in Fig. 2b in der zy-Ebene (senkrecht zur xy-Ebene) darstellt ist. In diesem Ausführungsbeispiel weist der Detektor 32 eine Vielzahl von Detektormodulen 32a bis 32g auf, die kreisförmig entlang eines Kreisbogens 34 angeordnet sind, wobei die Detektormodulen 32a bis 32g grundsätzlich den Detektormodulen 32a und 32b aus Fig. 1 entsprechen. In anderen Worten ausgedrückt heißt das, dass durch die kreisförmige Anordnung der Detektormodule 32a bis 32g ein gebogener Zeilendetektor ausgebildet wird, bei dem die Vielzahl der Detektormodule 32a bis 32g tangential auf der Kreisbahn 34 nebeneinander bzw. direkt aneinander angrenzend angeordnet sind, so dass über alle Erfassungsbereiche der Detektormodule 32a bis 32g die durch einen Emissionswinkel α₂₆ der Röntgenstrahlung 26 aufgespannte zylindermantelflächenförmige Erfassungsfläche erfasst werden kann. Hierdurch ergibt sich folglich, dass alle Flächennormalen, die zum Beispiel im Schwerpunkt der Detektormodulen 32a bis 32g angreifen, in einer sogenannten Normalenebene, die den Referenzbereich schneidet, angeordnet sind. Es sei ferner angemerkt, dass die einzelne Detektormodulen 32a bis 32g parallel zu der jeweiligen Tangente der Kreisbahn 34 angeordnet sein können, wobei bevorzugterweise die zu der jeweiligen Tangente gehörende Flächennormale den jeweiligen Erfassungsbereich in dem Flächenschwerpunkt des jeweiligen Detektormoduls 32a bis 32g schneidet.

Die Detektormodule 32a bis 32g können beispielsweise Mehrzeilendetektoren mit einer Vielzahl von Sensoren bzw. Pixeln in einer ersten Richtung s₁ und zweiten Richtung s₂ (jeweils senkrecht zu dem Kreisbogen 34) und/oder mit einer Vielzahl von Sensoren bzw. Pixeln in einer dritten Richtung t₁ und vierten Richtung t₂ (jeweils tangential zu dem Kreisbogen 34) sein. Es sei angemerkt, dass derartige Detektoren 32 in der Breite (vgl. erste und zweite Richtung s₁ bzw. s₂) im Vergleich zu ihrer Länge (vgl. dritte und vierte Richtung t₁ bzw. t₂, also von dem Detektormodul 32a zu dem Detektormodul 32g) sehr schmal sind (z.B. wenige Pixel), so dass es typischerweise ausreichend ist, den Detektor 32 entlang der Längsrichtung (vgl. dritte und vierte Richtung t₁ bzw. t₂) zu krümmen. Wie in Fig. 2b zu erkennen ist, sind die ersten und zweiten Richtungen s₁ bzw. s₂ und die dritte und vierte Richtungen t₁ bzw. t₂ (in der dargestellten Projektion) jeweils parallel zueinander, wobei die erste und zweite Richtung s₁ und s₂ gegenüber der dritten und vierten Richtungen t₁ und. t₂ senkrecht ist.

Für jeden dieser Detektormodule 32a bis 32g ist eine Flächennormale eingezeichnet, die sich senkrecht von der Oberfläche des jeweiligen Erfassungsbereichs erstreckt, wobei sich alle Flächennormalen der Detektormodule 32a bis 32g innerhalb des Referenzbereichs kreuzen oder sogar in einem gemeinsamen Schnittpunkt, der dem Brennfleck der Strahlungsquelle 27 entspricht, treffen. Insofern weist jede Flächennormale der Detektormodule 32a bis 32g eine Länge auf, die dem Radius des Kreisbogens 34 und damit auch einem Fokus-Detektor-Abstand a₂₇_₃₂ zwischen der Strahlungsquelle 27 und dem Röntgendetektor 32 entspricht. Also ist jedes einzelne Detektormodul 32a bis 32g gleich weit von der punktförmigen Strahlungsquelle 27 entfernt und senkrecht zu dieser angeordnet, so dass die Röntgenstrahlung 26 immer senkrecht auf das jeweilige Detektormodul 32a bis 32g oder genauer auf einen Mittelpunkt bzw. Schwerpunkt des jeweiligen Detektormoduls 32a bis 32g trifft. Es sei ferner angemerkt, dass die einzelnen Flächennormalen auf die Erfassungsebenen der Detektormodule 32a bis 32g alle senkrecht auf den Schwerpunkt der jeweiligen Erfassungsebenen des jeweiligen Detektormoduls 32a bis 32g gefällt sein können, wenn die einzelnen Detektormodulen 32a bis 32g längliche bzw. flache Detektormodule sind. Der jeweilige Flächenschwerpunkt entspricht dem Mittelpunkt der rechteckigen Erfassungsfläche der einzelnen Detektormodule 32a bis 32g oder dem Mittelpunkt des gewählten Erfassungsbereichs des jeweiligen Detektormoduls 32a bis 32g. Insbesondere beim industriellen Einsatz, z.B. in der Hochenergiebildgebung, bietet die dargestellte Vorrichtung 32 Vorteile, da hier diese Verfälschung in der Ortsauflösung durch die Schrägdurchstrahlung aufgrund der eingesetzten dicken Sensorschichten besonders hoch ist.

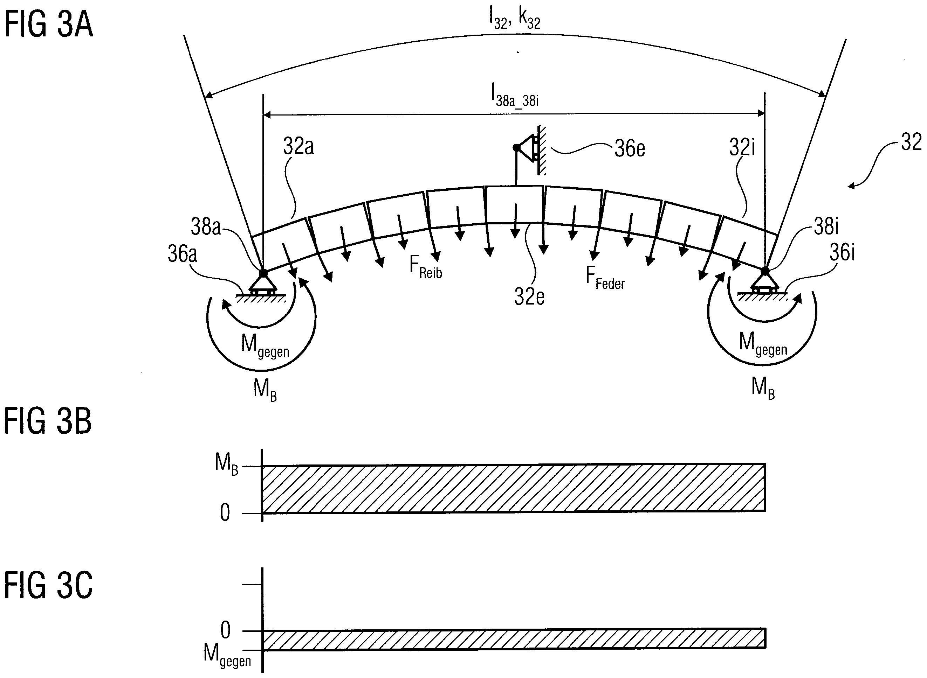

Analog zu dem Ausführungsbeispiel aus Fig. 1 sind die einzelnen Flächennormalen der Detektormodule 32a bis 32g bzw. der Radius des Kreisbogens 34 mittels der Manipulationseinrichtung (nicht dargestellt) einstellbar, so dass der Röntgendetektor 32 für unterschiedliche Abstände a_{27_32} adaptiert werden kann. Eine Möglichkeit, um die Ausrichtung der Flächennormalen der Detektormodule 32a bis 32g vorzunehmen, wird Bezug nehmend auf Fig. 3a bis 3c erläutert.

Fig. 3a zeigt den Röntgendetektor 32 mit neun Detektormodulen 32a bis 32i, die elastisch, z.B. mittels Federn miteinander verbunden sind. Hierdurch sind je zwei benachbarte Detektormodule der Detektormodule 32a bis 32i um einen gemeinsamen Drehpunkt bzw. eine gemeinsame Drehachse relativ zueinander drehbar. Die Drehachse kann beispielsweise durch Berührpunkte bzw. eine Berührachse der der Strahlungsquelle zugewandten Sensorschichtecken zweier benachbarter Detektormodule gebildet sein oder zwischen zwei Detektormodulen liegen. Des Weiteren ist der gesamte Röntgendetektor 32 so gelagert, dass die jeweiligen Detektormodule 32a bis 32i gemeinsam einstellbar sind. Hierzu ist das Detektormodul 32a mittels einer ersten Linearführung 36a und das letzte Detektormodul 32i mittels einer weiteren Linearführung 36i gelagert ist. An jeder Linearführung 36a und 36i ist der Röntgendetektor 32 gegenüber der Linearführung 36a und 36i rotorisch mittels einer Rotationslagerung 38a bzw. 38i gelagert. Über diese Rotationslagerungen 38a und 38i kann ein Biegemoment M_{b} z.B. mittels elektrischen (Stell-) Motoren, die eine Manipulationseinrichtung darstellen, in den Röntgendetektor 32 eingebracht werden.

Durch Einbringen des Biegemoments M_{b} in die als elastische Kette miteinander verbundenen Detektormodule 32a bis 32i verteilt sich das Biegemoment M_{b} gleichmäßig entlang der neun Detektormodule 32a bis 32i, wie in Fig. 3b dargestellt ist. Durch die gleichmäßige Verteilung des Biegemomentes M_{b} über eine gesamte Länge l₃₂ des Röntgendetektors 32 wird dieser kontinuierlich verbogen, so dass sich die einzelnen Detektormodule 32a bis 32i entlang einer Kreisbahn bzw. entlang eines Kreissegments k₃₂ anordnen, wobei der Kreisbogenradius (und somit die Anordnung der Flächennormalen) über das Biegemoment M_{b} einstellbar ist. Die konstante Verteilung des Biegemoments M_{b} über die Länge l₃₂ wird beispielsweise dadurch erreicht, dass zur Lagerung der einzelnen Detektormodulen 32a bis 32i Federn mit einer gleichen oder annährend gleichen Federhärte eingesetzt werden. Hieraus ergibt sich die Konsequenz, dass (bei Einleitung eines Biegemoments M_{b}) zwischen jedem Detektormodul und dem nächstliegenden Detektormodul 32a bis 32i bzw. zwischen zwei Flächennormalen benachbarter Detektormodule 32a bis 32i derselbe Winkel ausgebildet wird. Dem Biegemoment M_{b} wirkt ein Gegenmoment M_{gegen} entgegen, welches ebenfalls über die gesamte Länge l₃₂ gleichmäßig verteilt ist, wie in Fig. 3c dargestellt. Dieses Gegenmoment M_{gegen} resultiert aus der Federkraft F_{Feder} und aus der Reibkraft F_{Reib}, die bei Verbiegung des Röntgendetektors 32 zwischen den einzelnen Detektormodulen 32a bis 32i entstehen. Es sei angemerkt, dass die Momente M_{b} und M_{gegen} sowie die Kräfte F_{Feder} und F_{Reib} als Pfeile in der schematischen Darstellung von Fig. 3a illustriert sind. Ein bei der Verbiegung des Detektors 32 mittels des Biegemoments M_{b} entstehendes Abstützmoment M_{b}-M_{gegen} wird durch die zwei Linearführungen 36a und 36i aufgenommen.

Durch die Biegung ändert sich ferner ein effektive Lagerabstand l_{38a_38i} (Abstand Linearführungen 36a und 36i), was durch die Linearführungen 36a bzw. 36i ausgeglichen wird. Damit sich der Röntgendetektor 32 nicht in Folge des Biegemoments M_{b} verschiebt bzw. damit dieser zentriert bleibt, kann der Röntgendetektor 32 entsprechend weiteren Ausführungsbeispielen eine weitere Linearführung 36e umfassen, die das mittlere Detektormodul 32e parallel zu seiner Flächennormalen bzw. senkrecht zu den Linearführungen 36a und 36i führt. Somit wird eine exakte Fokussierung bzw. Ausrichtung des Fokus des Röntgendetektors 32 sichergestellt. Es sei ferner angemerkt, dass es für die Einstellung der Flächennormalen auch eine andere Lagerung und Krafteinleitung denkbar wären. Eine Alternative, den Röntgendetektor 32 zu verbiegen wäre es, den Abstand l_{38a_38i} direkt zu variieren bzw. zu verkürzen.

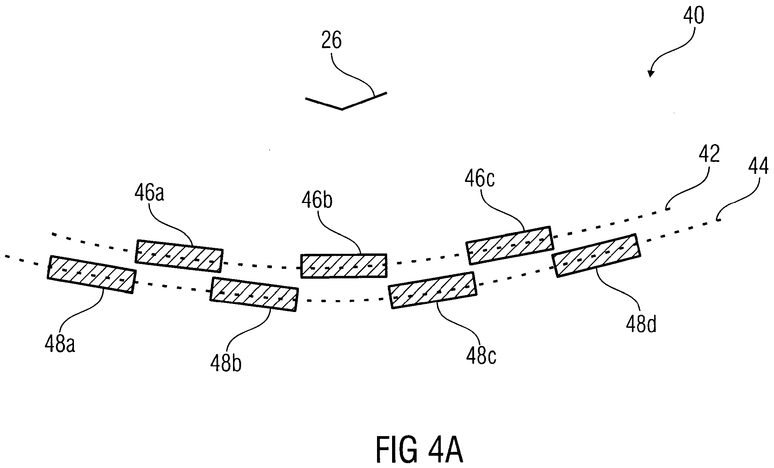

Fig. 4a zeigt ein weiteres Ausführungsbeispiel eines Röntgendetektors 40, bei dem die einzelnen Detektormodule auf zwei unterschiedlichen koaxialen Kreisbahnen 42 und 44 angeordnet sind. Auf der Kreisbahn 42, die den kleineren Radius der zwei Kreisbahnen 42 und 44 aufweist, sind drei Detektormodule 46a, 46b und 46c angeordnet. Im Gegensatz zu den oben diskutierten Ausführungsbeispielen sind hier die Detektormodule 46a, 46b und 46c nicht direkt aneinander anliegend bzw. angrenzend, sondern mit Zwischenräumen voneinander beabstandet angeordnet. Die Abstände bzw. Zwischenräume weisen näherungsweise die Breite der einzelnen Detektormodule 46a, 46b und 46c bzw. bevorzugterweise eine geringere Breite als die Detektormodule auf der zweiten Kreisbahn 44 auf. Auf dieser zweiten Kreisbahn 44 sind die vier Detektormodule 48a, 48b, 48c und 48d versetzt zu den Detektormodulen 46a, 46b und 46c, das heißt in den Zwischenräumen bzw. neben den Detektormodulen 46a, 46b und 46c angeordnet.

Durch diese versetzte Anordnung sind die nebeneinander liegenden Erfassungsebenen jeweils auf zwei unterschiedlichen Kreisbahnen 42 bzw. 44 angeordnet, so dass insgesamt mittels der Detektormodule 48a, 46a, 48b, 46b, 48c, 46c und 48d der gesamte Erfassungsbereich erfasst werden kann. Dieses Ausführungsbeispiel mit den auf koaxialen Kreisbahnen 42 und 44 mit unterschiedlichen Radien angeordneten Detektormodulen bietet den Vorteil, dass sich die einzelnen Erfassungsbereiche der Detektormodule 48a bis 48d bzw. 46a bis 46c überlappen, so dass keine "Nicht-Erfassungsbereiche" bzw. "toten Bereiche", beispielsweise verursacht durch die flexible Lagerung zwischen zwei benachbarten Detektormodulen, entsteht.

Infolge der koaxialen Anordnung treffen sich entsprechend den obigen Ausführungsbeispielen die jeweiligen Flächennormalen der Detektormodule 48a bis 48d und 46a bis 46c in dem Brennfleck der Strahlungsquelle (nicht dargestellt), wobei durch die zwei Radien der Kreisbahnen 42 und 44 kein einheitlicher Fokus-Detektor-Abstand besteht. Analog zu den obigen Ausführungsbeispielen sind die Radien der Kreisbahn 42 und 44 und damit die Flächennormalen zueinander bzw. der Winkel zwischen den Flächennormalen einstellbar. Ein derartiger Detektor 40 entspricht hinsichtlich Manipulationseinrichtung und Lagerung grundsätzlich dem in Fig. 3 diskutierten Aufbau, ist jedoch wesentlich komplexer.

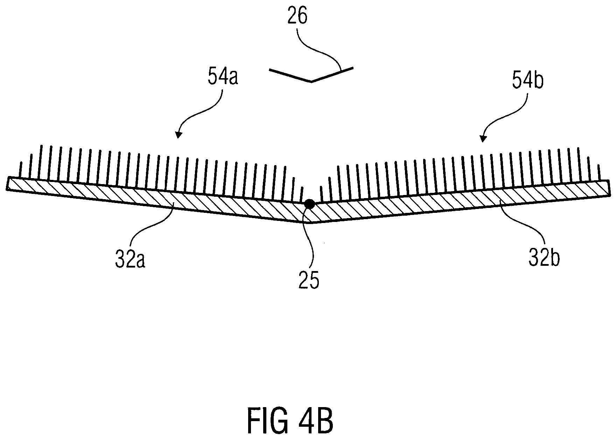

Fig. 4b zeigt entsprechend dem Ausführungsbeispiel aus Fig. 1 zwei auf einer Kreisbahn 32a und 32b angeordnete Detektormodule. Ebenso entsprechend dem Ausführungsbeispiel aus Fig. 1 sind die zwei Detektormodulen 32a und 32b um den Drehpunkt 25 drehbar gelagert. Im Gegensatz zu dem Ausführungsbeispiel aus Fig. 1 weisen die Detektormodule 32a und 32b auf Seite der Erfassungsbereiche, also auf der der Strahlung 26 zugewandten Seite jeweils ein Streustrahlraster 54a bzw. 54b auf. Die Streustrahlenraster 54a und 54b umfassen parallel angeordnete Bleche, die sich von der jeweiligen Erfassungsebene senkrecht, also in Richtung der Flächennormalen erstrecken. Die Streustrahlenraster 54a und 54b können entweder in einer ersten und zweiten Richtung (senkrecht zu den jeweiligen Flächennormalen und senkrecht zu der ersten bzw. zweiten Richtung) oder entlang der dritten und vierten Richtung (tangential zu der Kreisbahn und senkrecht zu der jeweiligen Flächennormalen) verlaufen. Die Streustrahlenraster 54a und 54b weisen Bleche bzw. Lamellen aus hoch absorbierendem Material, wie z.B. Blei oder Wolfram, auf, wobei in den Zwischenräumen ein für Röntgenstrahlung nahezu transparentes Filmmaterial vorhanden sein kann.

Durch die Unterteilung des gesamten Detektors 32 in mehrere Detektormodule 32a und 32b ist der genutzte Raumwinkel pro Detektormodul 32a bzw. 32b klein. Die Streustrahlenraster 54a und 54b schränken den genutzten Raumwinkel nicht weiter ein, so dass die Strahlung (selbst in Randbereichen der Detektormodule 32a und 32b) nahezu senkrecht auftrifft und Streustrahlung herausgefiltert werden kann, was die Bildqualität bzw. erreichbare Kontrastsensitivität erhöht. Durch die veränderbare relative Anordnung der Detektormodule 32a und 32b können die Streustrahlenraster 54a und 54b für alle Detektormodule 32a und 32b immer senkrecht zu den jeweiligen Bilderfassungsebenen angeordnet werden, was gegenüber den üblichen Streustrahlenrastern, die gewinkelt auf den Fokuspunkt der Röntgenstrahlung ausgerichtet angeordnet werden müssen, eine erhebliche Reduzierung des Fertigungsaufwands darstellt. Somit sind derartige Streustrahlenraster 54a und 54b auch bei industrieller Nutzung von Röntgensystemen einsetzbar, da diese nicht mehr wie sonst üblich den Detektor auf einen bestimmten Fokus-Detektor-Abstand einschränken.

Da der Einfluss der vertikalen Streustrahlung gering ist, wird bevorzugterweise das Streustrahlungsraster 32a und 32b nur in der ersten bzw. zweiten Richtung, also senkrecht zur Kreisbahn angeordnet, um den Füllfaktor der Pixel möglichst groß zu halten bzw. eine minimale Abschattung zu verursachen. Ursächlich für den größeren Einfluss der horizontalen Streustrahlung sind die Bezug nehmend Fig. 2 erläuterten Abmessungen der Detektormodule 32a und 32b.

Da der Drehmittelpunkt 25 der Detektormodule 32a und 32b an der vorderen Kante der Sensormodule 32a und 32b liegt, würden die Streustrahlenraster 54a und 54b den Bewegungsbereich der Detektormodule 32a und 32b einschränken. Um dies zu vermeiden, werden die Streustrahlenraster 54a und 54b entsprechend weiteren Ausführungsbeispielen zum Detektorrand bzw. zur Drehachse 25 hin abgeschrägt. Es sei angemerkt, dass durch die Abschrägung der Anteil an der detektierten Streustrahlung in den Randbereichen leicht zunehmen kann, wobei diese Effekte eben auf wenige Pixel im Randbereich beschränkt sind und gering ausfällt.

Hinsichtlich der Anordnung der Streustrahlenraster 54a und 54b in der ersten und zweiten bzw. dritten und vierten Richtung wäre es alternativ möglich, dass die Streustrahlenraster 54a und 54b sowohl in der ersten als auch in der dritten Richtung bzw. sowohl in der zweiten als auch in der vierten Richtung angeordnet sind und so ein zweidimensionales Karoraster bilden.

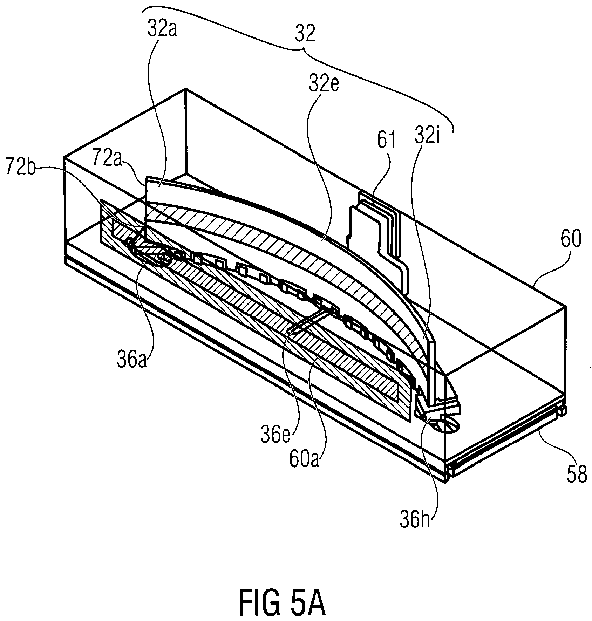

Bezug nehmend auf Fig. 5a bis 5e wird ein Konstruktionsbeispiel eines Röntgendetektors 32 beschrieben, wobei dieser Röntgendetektor 32 grundsätzlich dem in Fig. 3 gezeigten Röntgendetektor 32 entspricht.

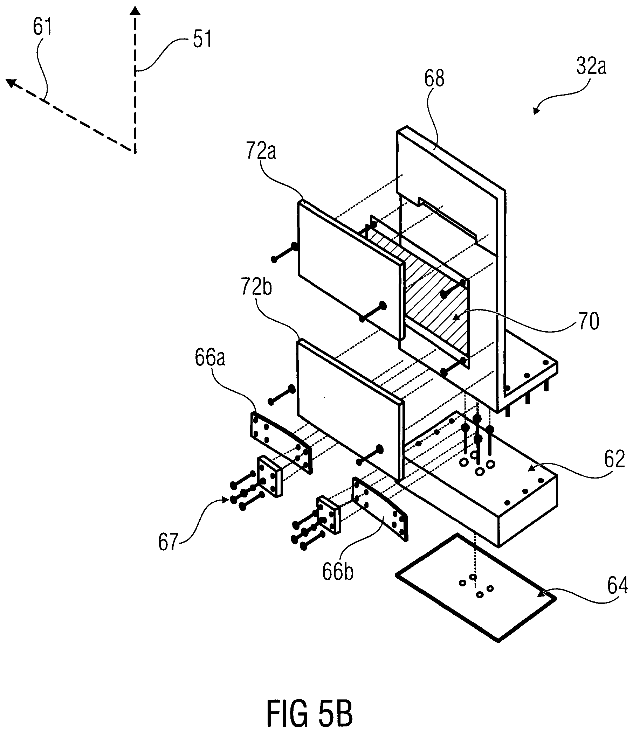

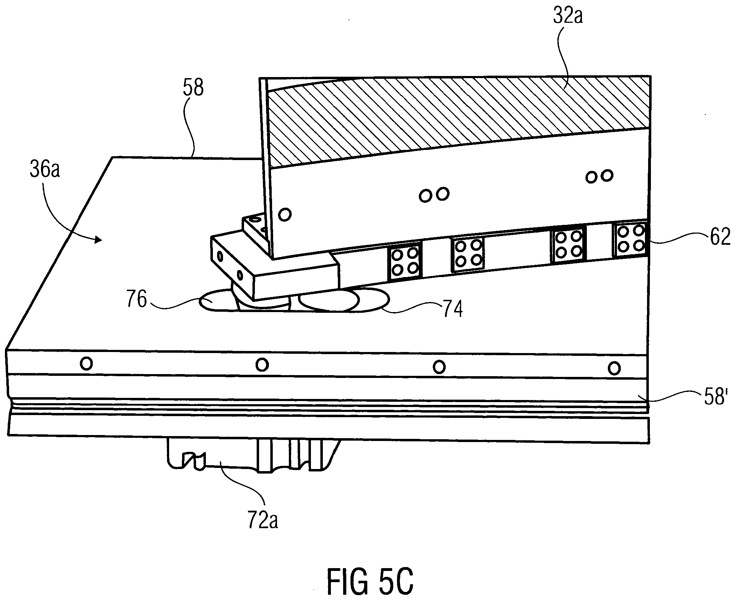

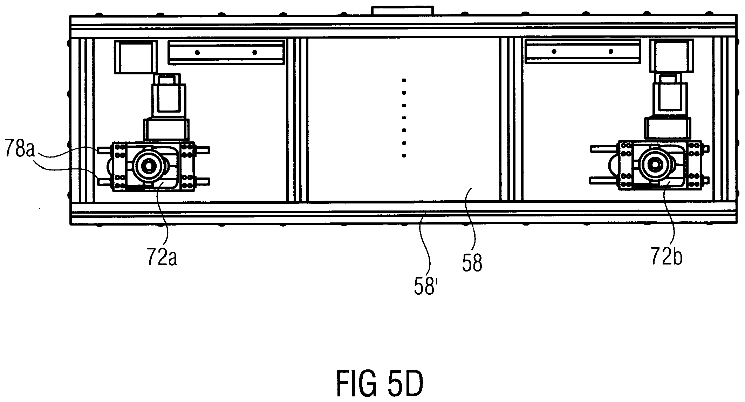

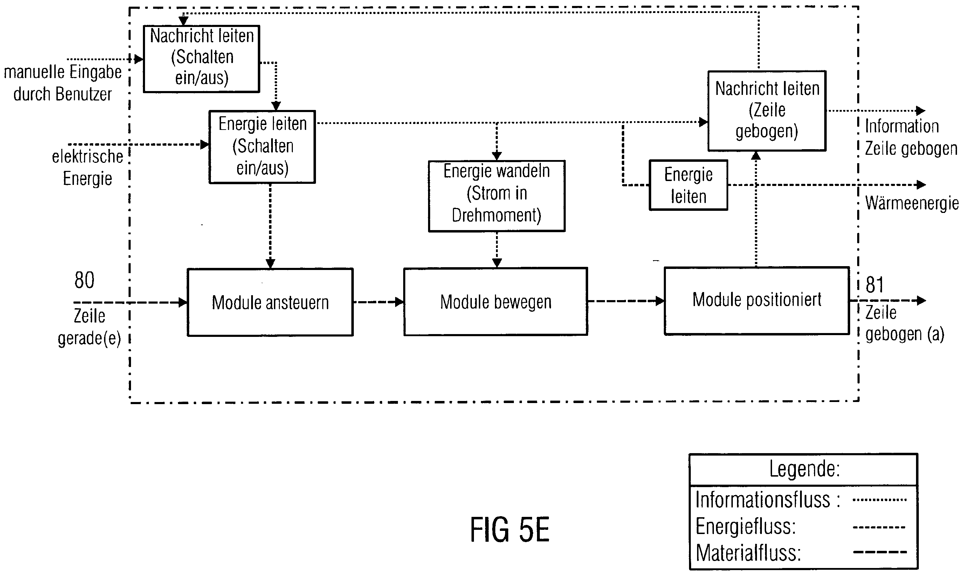

Der in Fig. 5 dargestellte Röntgendetektor 32 weist eine Vielzahl von flexibel miteinander verbundenen Detektormodulen 32a bis 32i, bevorzugt in einer ungeraden Anzahl auf. Die Vielzahl von Detektormodulen 32a bis 32i sind auf einer Grundplatte 58 angeordnet, die beispielsweise mittels Aluminiumdruckguss hergestellt ist. Jedes dieser Detektormodule 32a bis 32i weist unterschiedliche Elemente, wie z.B. eine Sensorplatine, einen Kollimator und/oder eine Verbindungsfeder auf, wie Bezug nehmend auf Fig. 5b detailliert erläutert wird. Die Vielzahl der relativ zueinander gelagerten Detektormodule 32a bis 32i, welche durch die drei Linearlager 36a, 36i und 36e gegenüber der Grundplatte gelagert sind, sind entlang eines Kreisbogens angeordnet, wobei der Radius durch eine elektrische Manipulationseinrichtung, welche beispielsweise zwei Elektromotoren aufweisen kann, veränderbar ist. Die Manipulationseinrichtung wird detailliert Bezug nehmend auf Fig. 5d erläutert, wobei die Lagerung 36a und 36i Bezug nehmend auf Fig. 5c näher ausgeführt wird.

Mittels der Manipulationseinheit wird entsprechend dem obigen Ausführungsbeispiel aus Fig. 3 ebenfalls das Drehmoment M_{b} an den äußeren Enden, das heißt an den Detektormodulen 32a und 32i, die mittels den Linearlager 36a und 36i geführt sind, eingeleitet. Die Linearlager 36a und 36i sind so angeordnet, dass die Detektormodule 32a und 32i längs zur Grundplatte beweglich sind, wobei das Linearlager 36e so angeordnet ist, dass das mittlere Detektormodul 32e quer, also entlang seiner Flächennormalen beweglich ist. Analog zu dem Ausführungsbeispiel aus Fig. 3 dienen die Linearlager 36a und 36i dazu, ein beispielsweise an dem Punkt der Linearlager 36a und/oder 36i eingebrachtes Drehmoment M_{b} /2 abzustützen, während das Linearlager 36e dazu dient, dass sich der Fokus des Röntgendetektor 32 nicht seitwärts (beispielsweise in Folge eines eingeleiteten Drehmoments M_{b}) gegenüber der Röntgenquelle (nicht dargestellt) verschiebt. In anderen Worten ausgedrückt heißt das, dass das Linearlager 36e das Detektormodul 32e (und damit den gesamten Röntgendetektor 32) so lagert, dass es sich nicht in Längsrichtung, sondern nur in Querrichtung entlang seiner Flächennormalen verschieben kann. Deshalb ist es dienlich, dass der Röntgendetektor 32 eine ungerade Anzahl an Detektormodulen aufweist, damit dieser in der Mitte bzw. durch dessen mittleres Detektormodul 32e zentral geführt wird.

Entsprechend weiteren Ausführungsbeispielen kann der in Fig. 5a dargestellte, auf der Grundplatte 58 montierte Röntgendetektor 32 auch ein Gehäuse 60 aufweisen, das als Deckel auf die Grundplatte 58 montiert ist. Das Gehäuse 60 dient dazu, um die Sensorplatinen und die Gleitflächen vor Staub und Schmutz zu schützen. Um dennoch eine uneingeschränkte Detektion für Röntgenstrahlung zu ermöglichen, weist das Gehäuse 60 einen strahlungsdurchlässigen Bereich bzw. ein Sichtfenster 60a auf, welches sich entlang dem gesamten Erfassungsbereich des Röntgendetektors 32 (auf der der Röntgenquelle zugewandten Seite) erstreckt und beispielsweise Kaptonfolie, CFK oder ein dünnes Metallblech aufweisen kann. Entsprechend weiteren Ausführungsbeispielen kann zur Klimatisierung des Röntgenmoduls ein Kühlsystem 61 an dem Gehäuse 60 vorgesehen sein.

Fig. 5b zeigt eines der Detektormodule 32a bis 32i als Explosionsdarstellung. Das dargestellte Detektormodul 32a weist einen Modulträger 62 auf, der mittels einer Platte 64 (z.B. Pennaglide™-Platte oder Glycodur™-Platte), die beispielsweise Polytetrafluorethylen (Teflon™) zur Reibungsminimierung umfasst, verschiebbar auf der Grundplatte (nicht dargestellt) gelagert ist. An dem Modulträger 62 sind ferner zwei Federn 66a und 66b befestigt. Die zwei Federn 66a und 66b sind an zwei gegenüberliegenden Seiten des Modulträgers 62 befestigt, um sowohl zu der einen als auch zu der anderen Seite eine flexible Verbindung mit dem benachbarten Detektormodul herzustellen. Diese Federn 66a und 66b übernehmen die Funktion eines Scharniers und dienen der Drehmomentübertragung. Darüber hinaus wird angemerkt, dass die Federn bzw. Verbindungsfedern 66a und 66b in diesem Ausführungsbeispiel auf der Innenseite des bogenförmig biegbaren Röntgendetektors 32 angeordnet sind, was es ermöglicht, den so genannten "toten Bereich" zu minimieren. Optional wäre es auch denkbar, dass weitere Federn, wie z.B. Schraubenfedern, zusätzlich zur Stabilisierung eingesetzt werden.

Auf dem Modulträger 62 ist ein Sensorplatinenträger 68 montiert, an dem eine Sensorplatine 70 befestigt ist. Ebenso sind an diesem Sensorträger 68 zwei Kollimatoren vor dem Erfassungsbereich der Sensorplatine 70 optional vorgesehen, die zur Erzeugung eines parallelen Strahlenverlaufs der auf die Sensorplatine 70 einfallenden Röntgenstrahlung dienen. Wie Bezug nehmend auf Fig. 5a dargestellt, kann der Kollimator in den Randbereichen der Sensorplatine 70 angeordnet sein, so dass nur die Sensorfläche bestrahlt wird. Sämtliche Detektorelektronik wird zum Schutz vor Röntgenbestrahlung hinter den Kollimatoren angeordnet.

Fig. 5c zeigt eine Detailaufnahme einer Manipulationseinrichtung 72a zusammen mit der Linearführung 36a, die an dem ersten Detektormodul 32a vorgesehen ist. Die Manipulationseinrichtung 72a ist unterhalb der Grundplatte 58 angeordnet und überträgt das Drehmoment mittels einer Welle 76 in den Modulträger 62 des Detektormoduls 32a. Da sich bei Einleitung eines Drehmoments mittels der Manipulationseinrichtung 72a der Krümmungsradius und damit die Längsposition des Detektormoduls 32a ändert, ist die Welle 76 mittels eines Längslochs 74 durch die Grundplatte 58 geführt. Über die Länge des Langlochs 74 sind die Längsbewegung und damit der kleinstmögliche Krümmungsradius begrenzt. Diese Linearführung 36a wird im Detail in Fig. 5d illustriert.

Fig. 5d zeigt die Grundplatte 58 von unten, wobei hier die Elektromotoren der Manipulationseinrichtung 72a sowie einer Manipulationseinrichtung 72b (an der Stelle der Linearführung 36i) dargestellt sind. Nachfolgend wird die Manipulationseinrichtung 72a stellvertretend für die Manipulationseinrichtung 72b erläutert. Die Manipulationseinrichtung 72a ist mittels zweier paralleler, längs zur Grundplatte 58 angeordneter Schienen 78a auf der Unterseite der Grundplatte 58 befestigt. Durch die Längsausrichtung der Schienen 78a ist einerseits die Manipulationseinrichtung 72a und damit das Röntgenmodul 32a längs verschiebbar bzw. längs geführt und andererseits kann durch die Schienen 78a das Drehmoment der Manipulationseinrichtung 72a abgestützt werden. Die Schienen 78a können beispielsweise als Schwalbenschwanzschienen ausgeführt sein. Es sei ferner angemerkt, dass die Grundplatte 58 mit einem Rahmen 58' verstärkt sein kann, um Durchbiegungen derselben zu vermeiden.

Fig. 5e zeigt ein Flussdiagramm zur Illustration der Einstellung des Krümmungsradius des flexiblen Röntgenmoduls 32. Im Ausgangszustand 80 wird von einem "nicht-gebogenen, (d.h. geraden) Röntgenmodul" ausgegangen. Um diesen Zustand in den erwünschten Endzustand "Röntgenmodul gebogen" 81 zu versetzen, werden die Module angesteuert und bewegt, bis diese positioniert sind. Hierzu erfolgt eine manuelle Eingabe durch den Benutzer, der die vorgegebene Endposition bzw. den Endzustand 81 vorgibt. Bei Eingabe werden die Elektromotoren so lange angesteuert bzw. mit elektrischer Energie versorgt, bis die Endposition 81 erreicht wird. Hierbei kann entsprechend weiteren Ausführungsbeispielen der Röntgendetektor einen Positionssensor aufweisen, der an die Steuerung die Information übermittelt, dass die Zeilen gebogen sind bzw. die Endposition erreicht ist. Daraufhin wird die elektrische Energie und damit das Drehmoment M_{b} für Elektromotoren nicht weiter erhöht, so dass die erreichte Endposition 81 gehalten wird, wobei die Endposition 81 prinzipiell durch ein Drehmomentgleichgewicht (M_{b} - M_{gegen} = 0) definiert ist, bei dem das mittels der Elektromotoren eingebrachte Drehmoment M_{b} dem insbesondere durch die Federn erzeugten Drehmoment M_{gegen} entspricht. Die Information, dass die Endposition erreicht ist, wird auch an den Benutzer ausgegeben. Entsprechend weiteren Ausführungsbeispielen kann die Manipulationseinrichtung auch Schrittmotoren umfassen, die bei Erreichen der Endposition abgeschaltet und hier fixiert werden.

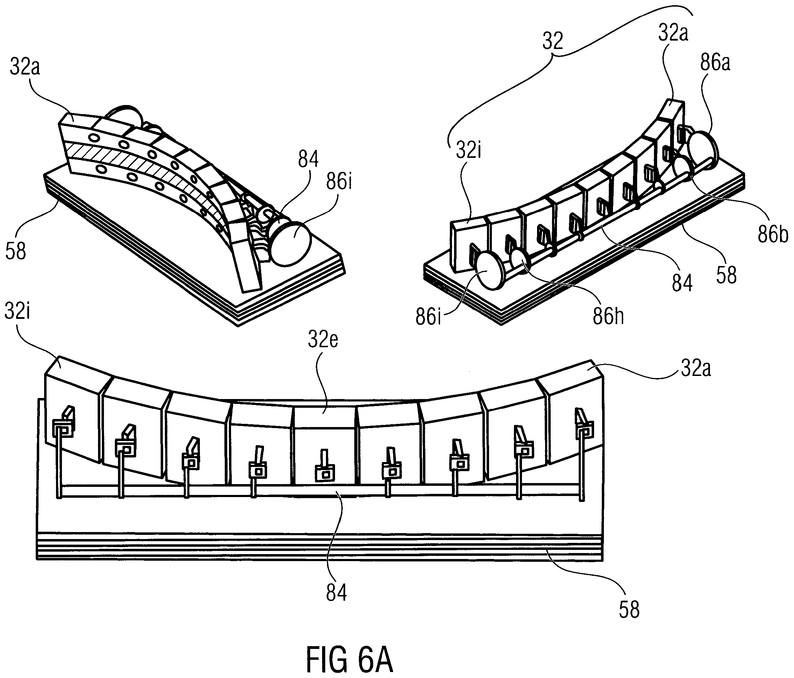

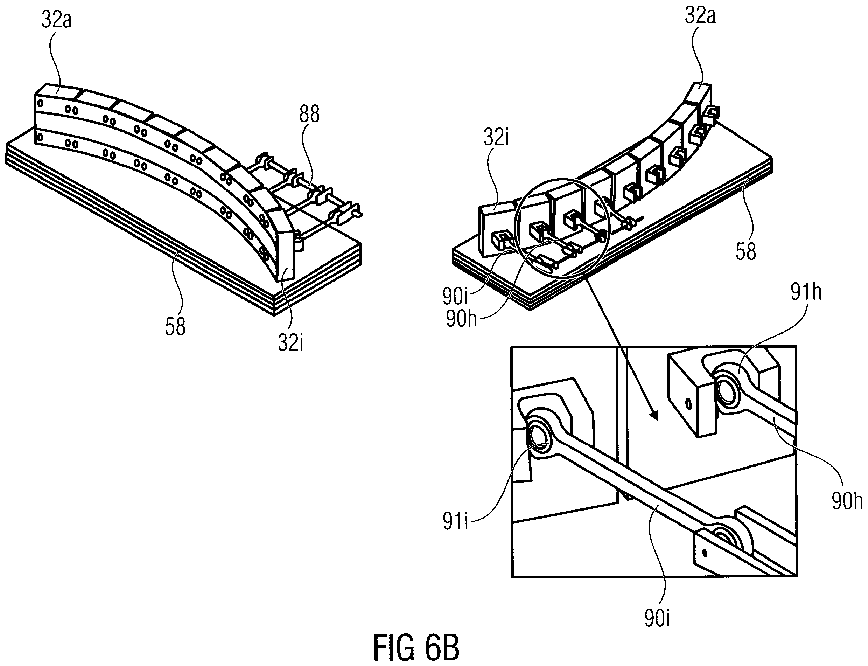

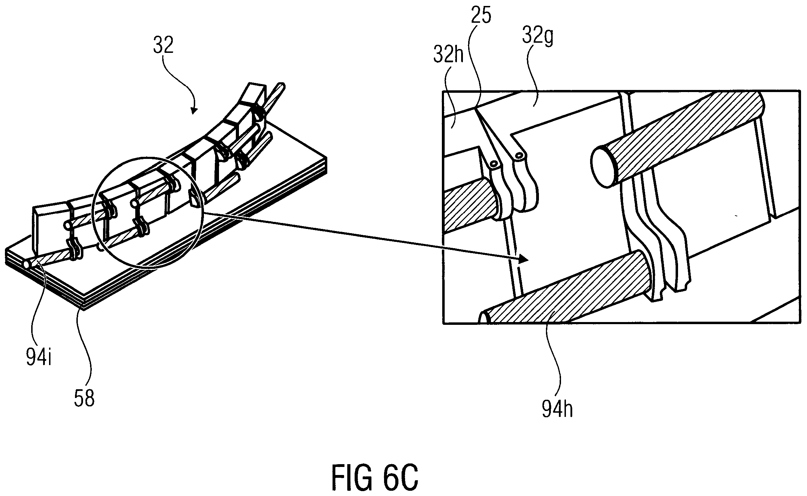

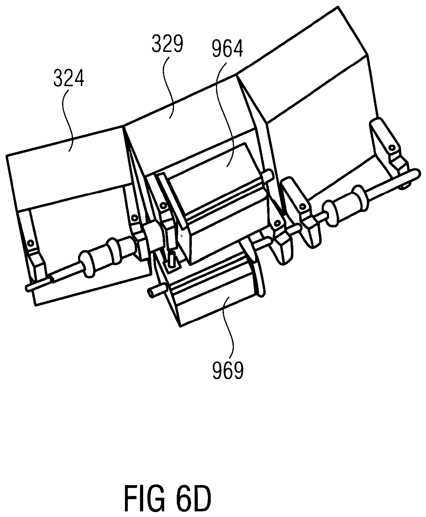

Bezug nehmend auf Fig. 6 werden im Folgenden verschiedenen alternative Manipulationseinrichtungen erläutert.

Fig. 6a zeigt drei Ansichten des Röntgendetektors 32, der auf der Grundplatte 58 angeordnet ist. Der Krümmungsradius des Röntgendetektors 32 wird hier durch eine Manipulationseinrichtung 84, die als Exzenterwelle 84 ausgeführt ist, realisiert. Die Exzenterwelle 84 ist entlang dem Röntgendetektor 32 auf der Rückseite desselben angeordnet und weist je Detektormodul 32a bis 32i unterschiedlich große Exzenterscheiben 86a bis 86i auf, die entweder als exzentrische (d.h. ovale) Scheiben oder als kreisrunde, aber exzentrisch (im Randbereich) aufgehängte Scheiben ausgeführt sind. Die am weitesten außen liegenden Exzenterscheiben, also 86a und 86i für die Detektormodule 32a und 32i, sind am größten, also größer als die weiter innen liegenden Exzenterscheiben 86b und 86h für die Detektormodule 32h und 32b, wobei das mittlere Detektormodul 32e keine Exzenterscheiben aufweist. In anderen Worten sind die Exzenterscheiben 86a und 86i hinsichtlich ihrer Position und ihrer relativen Größe zueinander entsprechend dimensioniert.

Da die Exzenterscheiben 86a bis 86i mit den Detektormodulen 32a bis 32i in Eingriff sind, erfolgt bei einer Drehung der Exzenterwelle 84 eine Verschiebung der Detektormodule 32a bis 32i (quer zu der Grundplatte 58). Je Detektormodul 32a bis 32i ist die Verschiebung unterschiedlich groß, da die Exzenterscheiben 86a bis 86i unterschiedliche Durchmesser aufweisen. Hierbei werden alle Detektormodule 32a bis 32i mit Ausnahme des mittleren Detektormoduls 32e verschoben, da dessen Flächennormale immer auf den Brennpunkt des Strahlungsquelle gerichtet ist (solange der Röntgendetektor 32 gegenüber der Strahlenquelle richtig positioniert ist). Durch die zur Mitte hin kleiner werdenden Durchmesser der Exzenterscheiben 86a bis 86i erfolgt eine Anordnung der Detektormodulen 32a bis 32i entlang einer Kreisbahn, wobei infolge der elastischen Verbindung der Detektormodule 32a bis 32i dieselben verdreht werden. Die Verschiebung und damit die Verdrehung sind hierbei von der Winkelposition der Exzenterwelle 84 abhängig. Entsprechend weiteren Ausführungsbeispielen können die Detektormodule 32a bis 32i auch auf der Rückseite einen Eingriffnahmeabschnitt, der gegebenenfalls mittels eines Gelenks ausgeführt ist, aufweisen.

Fig. 6b zeigt drei Ansichten des auf der Grundplatte 58 angeordneten Röntgendetektors 32, der in diesem Ausführungsbeispiel mit einer Manipulationseinrichtung 88 verstellt wird. Die Manipulationseinrichtung 88 weist eine Kurbelwelle 88 mit unterschiedlich langen Stößeln (Pleuelstangen) 90a bis 90i auf. Entsprechend dem Ausführungsbeispiel aus Fig. 6a, sind die außen liegenden Stößel gegenüber den weiter innen liegenden Stößeln länger. Also ist der Stößel 90i, der mit dem Detektormodul 32i verbunden ist, länger als der Stößel 90h, der mit dem Detektormodul 32h verbunden ist, was bei einer Veränderung der Winkelposition der Kurbelwelle 88 in einer weiter ausgedehnten Verschiebung auf der Grundplatte 58 bzw. in einer größeren Verdrehung des Detektonnoduls 32i resultiert (vgl. Fig. 6a). Wie in der Vergrößerungsdarstellung der Stößel 90i und 90h gezeigt ist, sind die Stößel mit den Detektormodulen mittels Kugelköpfen 91i bzw. 91h gelenkig verbunden. Es sei ferner angemerkt, dass die Kröpfungen der Kurbelwelle 88 auch unterschiedlich groß dimensioniert sein können.

Bezug nehmend auf Fig. 6a und 6b wird angemerkt, dass durch Ausrichtung der Detektormodule 32a bis 32i entlang eines Kreisbogens sowohl eine rotorische Bewegung der Detektormodule 32a bis 32i als auch eine translatorische Bewegung der Detektormodule 32a und 32i erfolgt. Die translatorische Bewegung wird durch die Manipulationseinrichtung 84 bzw. 88 direkt bewirkt, während die rotorische Bewegung, also das Einleiten des Biegemoments dadurch erfolgt, dass die einzelnen Detektormodule 32a bis 32i relativ zueinander gelagert sind.

Fig. 6c zeigt zwei Ansichten eines weiteren Ausführungsbeispiels einer Manipulationseinrichtung, wobei hier zwischen den einzelnen Detektormodulen 32a bis 32i des Röntgendetektors 32 linear veränderliche Elemente 94a bis 94i vorgesehen sind. Diese in ihrer Länge veränderlichen Elemente 94a bis 94i, die auch Linearaktoren genannt werden, sind auf der Rückseite, also mit einem Hebelarm gegenüber der Drehachse 25 (Stoßkante der zwei Detektormodule 32a und 32g) angeordnet.

Im Folgenden wird die Funktionsweise der linear veränderlichen Elemente 94a bis 94i anhand des zwischen den Detektormodulen 32g und 32h angeordneten linear veränderlichen Elemente 94h erläutert. Bei einer Längenänderung bzw. Krafteinleitung des linear veränderlichen Elements 94 erfolgt eine Drehmomenteinleitung in die zwei Linearmodule 32h und 32g, so dass hieraus eine Verkippung bzw. Verdrehung der zwei Detektormodule 32g und 32h resultiert. Da jeweils zwischen zwei Detektormodulen die Linearaktoren vorgesehen sind, können alle Detektormodule 32a und 32g wiederum entlang der Kreisbahn angeordnet werden.

Fig. 6d zeigt eine weitere mögliche Ausführungsform der in Fig. 6c beschriebenen Manipulationseinrichtung. Hierbei ist zwischen zwei Detektormodulen 32g und 32h ein linear veränderliches Element 96h angeordnet, das einen Schrittmotor und eine Spindel mit einer Balgkupplung aufweist. Der an dem einen Detektormodule 32g angeordnete Schrittmotor verändert bei Rotation über die Spindel, die mit dem anderen Detektormodul 32h über ein Gewinde in Eingriff ist, den Abstand und damit die Verkippung der zwei Detektormodule 32g und 32h. Die optional vorgesehene Balgkupplung verhindert hierbei Verspannungen im Gewinde, die durch die Verkippung der zwei Detektormodule 32g und 32h entstehen können.

Bezug nehmend auf Fig. 6a bis 6d sei angemerkt, dass die Exzenterwelle 84, die Kurbelwelle 88 sowie die linear veränderlichen Elemente 94a bis 94i und die linear veränderlichen Elemente 96a bis 96i sowohl elektrisch, z.B. mittels eines Elektro- oder Schrittmotors, als auch mechanisch, z.B. mittels einer Handkurbel, angesteuert werden können. Ebenso wäre es denkbar, dass die Winkelverstellung bzw. Krümmungsradiusverstellbar mittels eines pneumatischen oder hydraulischen Systems (z.B. pneumatisch angesteuerte Linearaktoren) realisiert sein kann. Ebenso sei angemerkt, dass auch andere Formen der Drehmomenteinleitung z.B. mittels eines Stecksystems (welches modular aufgebaute Abstands- bzw. Winkelelemente umfasst) ausgeführt sein kann.

Bezug nehmend auf Fig. 5b sei ferner angemerkt, dass die Federn 66a und 66b auch durch einfache Scharniere oder durch andere biegbare Elemente realisiert sein können, wobei die Verbindung zwischen zwei benachbarten Detektormodulen bevorzugterweise spielfrei ist. Ebenso wäre es alternativ denkbar, dass anstelle des Permaglidepads 64 auch Rollen oder eine andere reibungsminimierte Lagerung zum Einsatz kommen.

Es sei ferner angemerkt, dass die Bezug nehmend auf Fig. 4b, 5a bis 5e und 6a bis 6d erläuterten Aspekte ebenso auf das Ausführungsbeispiel aus Fig. 4a angewendet werden können, bei dem die Detektormodule auf unterschiedlichen koaxialen Kreisbahnen, also versetzt zueinander angeordnet sind.

Nachfolgend wird die Erfindung zusammenfassend mit anderen Worten beschrieben: Es wurde eine Methode entwickelt, um die Vorteile eines gekrümmten Detektors auch in einem flexiblen industriellen Röntgensystem einsetzen zu können, welche eine flexible Einstellung des Krümmungsradius ermöglicht. Das man den Krümmungsradius flexibel einstellen kann, stellt die Hauptinnovation dieser Erfindungsmeldung dar. Außerdem ermöglicht der Aufbau den einfachen Einsatz eines Streustrahlenrasters. Das Konzept ist dabei für eine große Anzahl an Modulen geeignet. Dies soll anhand eines Beispielsystems erläutert werden. Die Anordnung erfolgt dabei, wie die Abbildung 2 zeigt, ähnlich der in üblichen Systemen, jedoch sind die einzelnen Detektormodule beweglich gelagert und über eine flexible mechanische Verbindung miteinander verbunden. Bei diesem Konzept kann der Radius z.B. über zwei Motoren und drei Linearführungen eingestellt werden, wie in Fig. 3a-3c gezeigt ist.

Der modulare Aufbau der Detektormodule erfolgt mittels sogenannter Moduleinheiten. Diese werden in einer ungeraden Anzahl auf der Grundplatte angeordnet (vgl. Fig. 5a). Um nur sehr kleine Lücken (und damit möglichst keine toten Bereiche) im Detektor zu haben, ist es vorteilhaft, dass die Module möglichst nahe nebeneinander platziert sind (vgl. Fig. 1). Die Sensoren der jeweils aneinander angrenzenden Detektormodule werden so platziert, dass sie sich mit der Ecke der zur Strahlung hingewandten Seite nahezu berühren. Dieser Berührungspunkt ist dabei gleichzeitig der Drehmittelpunkt, um den sich die Module bei einer Verstellung des Krümmungsradius des Detektors bewegen. Mechanisch wird der Drehmittelpunkt dadurch genau an diesem Ort gelegt, dass die Feder hier ihren Knickpunkt besitzt und durch Abstandshalter richtig platziert wird.

Die Moduleinheiten befinden sich auf einer Grundplatte aus Aluminiumguss. Zur Reibungsminimierung wird eine Platte aus Permaglide (Polytetrafluorethylen (PTFE)) unter dem Modulträger befestigt. Die Modulträger werden mit Hilfe der Verbindungsfedern miteinander verbunden. Neben dem in der Explosionszeichnung gezeigten Aufbau, ist auch der Einsatz einer weiteren Feder, zur zusätzlichen Stabilisierung, oberhalb der Sensorfläche möglich. Die Federn übernehmen die Funktion eines Scharniers und dienen der Drehmomentübertragung. Das Drehmoment wird über zwei Antriebe an den äußeren Enden eingeleitet. Die Antriebe werden über Linearführungen geführt. Die Moduleinheit in der Mitte wird ebenfalls mit einer Linearführung geführt. Das Biegen der Verbindungsfeder mit einem gleichmäßigen Biegemoment ermöglicht die Einstellung eines Kreisbogens unter Verwendung von nur zwei Motoren.

Die Antriebe befinden sich unter der Grundplatte und werden ebenfalls durch Linearführungen geführt. Um eine Durchbiegung der Grundplatte zu verhindern, wird die Grundplatte mit einem Rahmen verstärkt. Um die Sensorplatine und die Gleitflächen vor Staub und Schmutz zu schützen, wird der Aufbau mit einer Umhausung verkleidet. Diese hat ein strahlendurchlässiges Fenster, wie z.B. Kaptonfolie, CFK oder ein dünnes Metallblech und verfügt über ein Kühlsystem.

Auch ein Detektoraufbau komplett ohne Lücken wäre umsetzbar. Hierfür wäre z.B. eine Modulanordnung in zwei Reihen vorteilhaft, wie in Fig. 4a gezeigt ist. Solch ein Detektor wäre jedoch im mechanischen Aufbau komplexer. Zudem besitzt er durch die zwei "Lagen" keinen einheitlichen Radius bzw. Fokus-Detektor-Abstand mehr.

Das hier entwickelte Konzept, bei dem jedes Detektormodul senkrecht auf den Röntgenquellort ausgerichtet ist, ermöglicht nun aber auch eine einfach umsetzbare Form des Streustrahlenrasters, welches sich gleichzeitig flexibel einsetzen lässt. Fig. 4b zeigt den Aufbau eines Streustrahlenrasters in der Draufsicht. Das Streustrahlenraster besteht aus parallel angeordneten Blechen. Zwischen diesen Blechen kann ein, für Röntgenstrahlung nahezu transparentes, Füllmaterial vorhanden sein. Die Bleche selbst sollten aus einem hochabsorbierenden Material wie Blei oder Wolfram bestehen. Diese einfache parallele Anordnung ist möglich, da der genutzte Raumwinkel pro Detektormodul sehr gering ist und deshalb die Strahlung auch an den Randbereichen des Moduls noch fast senkrecht auftrifft. Durch den gekrümmten Aufbau ist diese Situation für jedes Detektormodul, unabhängig von dessen Position, gleich.

Das Streustrahlenraster absorbiert jedoch nicht nur Streustrahlung, sondern auch einen Teil der Primärintensität. Da das Streustrahlenraster einen Teil der aktiven Pixelfläche bzw. des Szintillators verdeckt, nimmt dessen Füllfaktor ab. Der Anteil der bedeckten Pixelfläche hängt von der Dicke der absorbierenden Bleche ab. Diese Dicke ist wiederum abhängig von der Energie der Röntgenstrahlung.

Das hier entwickelte Konzept ist überwiegend für einzeilige oder mehrzeilige Detektoren und nicht für Flächendetektoren angedacht. Da der genutzte Raumwinkel bei diesen Detektoren zwar sehr breit aber in der Höhe flach ist, ist auch der Anteil der auf den Detektor auftreffenden Streustrahlung aus horizontaler Richtung damit wesentlich größer, als der aus vertikaler Richtung. Der Anteil der horizontal gerichteten Streustrahlung kann durch ein vertikales Raster abgefangen werden. Zum Abfangen der vertikalen Streustrahlung wäre ein zusätzliches horizontales Raster nötig, so dass ein Karoraster entstehen würde. Da der Einfluss der vertikalen Streustrahlung jedoch gering ist und um den Füllfaktor des Pixels möglichst groß zu halten, kann bevorzugt auf ein horizontales Raster verzichtet werden.

Da der Drehmittelpunkt zweier Detektormodule der vordere Eckpunkt der Sensoren ist, würden sich die Streustrahlenraster an den Detektormodulrändern berühren. Um dies zu vermeiden, werden diese zum Detektorrand hin abgeschrägt. Hierdurch steigt jedoch der Anteil an detektierter Streustrahlung in diesem Bereich leicht an. Dieser Effekt ist zum Detektormodulrand hin ansteigend. Dadurch nimmt die erreichbare Kontrastsensitivität zu den Modulrändern leicht ab. Bei üblichen Detektoraufbauten sollte sich der Effekt dabei auf wenige Pixel beschränken. Gegenüber einem Detektor ohne Streustrahlenraster bietet dieser Aufbau große Vorteile in der erreichbaren Kontrastsensitivität.

Der beschrieben Aufbau ennöglicht es den Krümmungsradius des Detektors anzupassen. Dies ermöglicht einen Einsatz eines fokussierten Detektors unter flexiblen Anforderungen. Durch die radiale Anordnung lässt sich die Schrägdurchstrahlung minimieren und ein einheitlicher Fokus-Detektor-Abstand für alle Detektormodule erreichen. Hierdurch werden die anfangs beschriebenen Nachteile aufgehoben und die Bildqualität, vor allem in den Randbereichen, im Vergleich mit einem geraden Zeilen- oder Flächendetektor, erheblich verbessert. Weiterhin ermöglicht der beschriebene Aufbau einen sinnvollen Einsatz eines Streustrahlenrasters, was die Bildqualität noch weiter steigern kann.

Durch den Einsatz der Moduleinheiten wird eine größtmögliche Flexibilität erreicht. Aufgrund der Verwendung von Verbindungsfedern wird eine frei einstellbare, gerade und radiale Anordnung mit nur zwei Antrieben ermöglicht.

Ihre typische Anwendung findet die Erfindung in Röntgendetektoren, welche in industriellen Röntgenprüfsystemen eingesetzt werden, die für die Prüfung unterschiedlicher Objekte vorgesehen sind. Die Erfindung lässt sich dabei sowohl in Röntgendetektoren mit nur einer Zeile, sogenannte Zeilendetektoren, also auch auf Detektoren mit vielen Zeilen, sogenannte Mehrzeilendetektoren, anwenden. Auf Grund der Modularität kann der Detektor dabei in seiner Länge den nötigen Anforderungen angepasst werden. Die Erfindung lässt sich sowohl für Röntgendetektoren niedriger Röntgenenergie, als auch für Röntgendetektoren hoher Röntgenenergie einsetzen.

Entsprechend weiteren Ausführungsbeispielen umfasst der Röntgendetektor eine Mehrzahl von zeilenförmigen Detektormodulen, die so angeordnet sind, dass der Röntgendetektor 20; 32 eine Zylindermantelfächensektion k32 mit einem einstellbaren Radius ausbildet.

Entsprechend weiteren Ausführungsbeispielen liegt die erste Flächennormale 22b in einem Flächenschwerpunkt des ersten Erfassungsbereichs 22a und die zweite Flächennormale (24b) in einem Flächenschwerpunkt des zweiten Erfassungsbereichs (24a).

Entsprechend einem Ausführungsbeispiel mit einem ersten und einem zweiten Röntgendetektor können die erste Kreisbahn 42 und die weitere Kreisbahn 44 koaxial angeordnet sein.

Entsprechend weiteren Ausführungsbeispielen sind die Röntgendetektormodule 20; 32; 32c-32i auf einer Platte 64, welche Polytetrafluorethylen oder andere Mittel zur Reibungsminimierung umfasst, verschiebbar gelagert.

Ein weiteres Ausführungsbeispiel weist einen Röntgendetektor 20; 32; 40 und eine Strahlungsquelle 27 auf, wobei der Referenzbereich in Abhängigkeit von einem Fokus-Detektor-Abstand a27_32 mittels der Manipulationseinrichtung positionierbar ist.

Bei einem weiteren Ausführungsbeispiel liegt ein Brennfleck der Strahlungsquelle 27 innerhalb des Referenzbereichs.

## Patentansprüche

1. Röntgendetektor (20; 32) mit folgenden Merkmalen:
einem ersten Detektormodul (32a) mit einem in einer ersten Erfassungsebene (22) liegenden ersten Erfassungsbereich (22a);
einem zweiten Detektormodul (32b) mit einem in einer zweiten Erfassungsebene (24) liegenden zweiten Erfassungsbereich (24a), das benachbart zu dem ersten Detektormodul (32a) ist; und
einer ersten Manipulationseinrichtung (72a), die ausgebildet ist, um die erste Erfassungsebene (22) des ersten Detektormoduls (32a) und die zweite Erfassungsebene (24) des zweiten Detektormoduls (32b) so zueinander auszurichten, dass sich eine erste Flächennormale (22b) der ersten Erfassungsebene (22) und eine zweite Flächennormale (24b) der zweiten Erfassungsebene (24) innerhalb eines Referenzbereichs kreuzen; und
einem weiteren Detektormodul (32c) mit einem in einer weiteren Erfassungsebene liegenden weiteren Erfassungsbereich, das benachbart zu dem zweiten Detektormodul (32b) ist und das gegenüber dem ersten und zweiten Detektormodul (32a, 32b) so angeordnet ist, dass die erste, zweite und weitere Erfassungsebene (22, 24) mit jeweiligen Flächenschwerpunkten tangential auf einer Kreisbahn (34) mit einem einstellbaren Radius verteilt sind; und
einer weiteren Manipulationseinrichtung (72b), die ausgebildet ist, um die weitere Erfassungsebene des weiteren Detektormoduls (32c) gegenüber der ersten und zweiten Erfassungsebene (22, 24) so auszurichten, dass sich die erste und zweite Flächennormale (22b, 24b) zusammen mit einer weiteren Flächennormalen der weiteren Erfassungsebene (24) innerhalb des Referenzbereichs kreuzen,
**gekennzeichnet dadurch, dass** das erste und zweite Detektormodul (32a, 32b) relativ zueinander durch eine erste Feder (66a) und das zweite (32b) und das weitere Detektormodul (32c) relativ zueinander durch eine zweite Feder (66b) verbunden sind,
wobei
a) die erste und zweite Feder (66a; 66b) eine gleiche Federhärte aufweisen und die Manipulationseinrichtungen (72a; 72b) ausgebildet sind, um einen Abstand (l₃₈ₐ__{38c}) zwischen dem ersten und weiteren Detektormodul (32a, 32i) zu variieren, und die Federn (66a) ausgebildet sind, um bei Variation des Abstands (l_{38a_38i}) den Röntgendetektor (20; 32) entlang der Kreisbahn (34) auszurichten,
oder
wobei
b) die Manipulationseinrichtungen (72a, 72b) ausgebildet sind, um das erste und/oder weitere Detektormodul (32c) mit einem Drehmoment (M_{b}) zu beaufschlagen, und die Federn (66a, 66b) ausgebildet sind, um das Drehmoment (M_{b}) gleichmäßig entlang aller Detektormodule (32a-32c) zu verteilen, um so die Detektormodule (32a-32c) entlang der Kreisbahn (34) auszurichten.

2. Röntgendetektor (20; 32) gemäß Anspruch 1, wobei die Manipulationseinrichtung (72, 72a, 72b) ausgebildet ist, um das erste und/oder weitere Detektormodul (32a-32i) mit einem Drehmoment (M_{b}) oder einer Kraft zu beaufschlagen, und wobei das erste, zweite und weitere Detektormodul (32a-32c) mittels Federn (66a, 66b) verbunden sind, die ausgebildet sind, um das Drehmoment (M_{b}) oder die Kraft gleichmäßig entlang aller Detektormodule (32a-32i) zu verteilen, um so die Detektormodule (32a-32i) entlang der Kreisbahn (34) auszurichten.

3. Röntgendetektor (20; 32) gemäß Anspruch 1 oder 2, wobei das erste, zweite und weitere Detektormodul (32a-32i) so miteinander gekoppelt sind, dass bei einer Einstellung des Radius ein einstellbarer Winkel zwischen der ersten und zweiten Flächennormalen einem einstellbaren Winkel zwischen der zweiten und weiteren Flächennormalen entspricht.

4. Röntgendetektor (20; 32) gemäß einem der vorherigen Ansprüche, wobei das erste Detektormodul (32a) eine Vielzahl von entlang einer ersten Richtung (s₁) angeordneten, zu einer ersten Detektorzeile gruppierten Sensorelemente aufweist und das zweite Detektormodul (32b) eine Vielzahl von entlang einer zweiten Richtung (s₂) angeordneten, zu einer zweiten Detektorzeile gruppierten Sensorelemente aufweist, wobei die erste und zweite Richtung (s₁, s₂) parallel zueinander sind.

5. Röntgendetektor (20; 32) gemäß einem der Ansprüche 1 bis 4, wobei das erste Detektormodul (32a) eine Vielzahl von in einer ersten und dritten Richtung (s₁, t₁) angeordneten Sensormodulen aufweist und das zweite Detektormodul (32b) eine Vielzahl von in einer zweiten und vierten Richtung (s₂, t₂) angeordneten Sensormodulen aufweist,
wobei die erste und zweite Richtung (s₁, s₂) parallel zueinander sind und die dritte und vierte Richtung (t₁, t₂) sich als Tangenten entlang der Kreisbahn (34) erstrecken.

6. Röntgendetektor (20; 32) gemäß einem der vorherigen Ansprüche, wobei das erste Detektormodul (32a) gegenüber dem zweiten Detektormodul (32b) so gelagert ist, dass zur Einstellung einer Ausrichtung der ersten und zweiten Flächennormalen (22b, 24b) das erste und zweite Detektormodul (32a, 32b) um eine Drehachse (25) relativ zueinander verdrehbar sind, und/oder
wobei die Drehachse (25) auf einer gemeinsamen Kante (25) des ersten und zweiten Detektormoduls (32a, 32b) auf Seite des ersten und zweiten Erfassungsbereichs (22a; 24a) gebildet ist; oder
wobei die Drehachse (25) zwischen und/oder parallel zu einer Kante (25) des ersten Detektormoduls (32a) auf Seite des ersten Erfassungsbereichs (22a) und einer Kante (25) des zweiten Detektormoduls (32b) auf Seite des zweiten Erfassungsbereichs (24a) gebildet ist.

7. Röntgendetektor (20; 32) gemäß einem der vorherigen Ansprüche, wobei das erste und zweite Detektormodul (32a, 32b) relativ zueinander durch ein bewegliches Verbindungselement(66a; 66b) gelagert sind, und
wobei das bewegliche Verbindungselement eine Feder (66a; 66b) mit einem definierten Knickpunkt ist, der ausgebildet ist, eine Drehachse (25) zu definieren, um welchen das erste und zweite Detektormodul (32a, 32b) relativ zueinander verdrehbar gelagert sind.

8. Röntgendetektor (20; 32) gemäß einem der Ansprüche 1 bis 7, wobei das erste Detektormodul (32a) ein Streustrahlungsraster (54a) entlang einer ersten Richtung (s₁) aufweist, das sich als Plattenelemente senkrecht von der ersten Erfassungsebene (22a) entlang der ersten Flächennormalen (22b) erstreckt, und das zweite Detektormodul (32b) ein Streustrahlraster (54b) entlang einer zweiten Richtung (s₂) aufweist, das sich als Plattenelemente senkrecht von der zweiten Erfassungsebene (24a) entlang der zweiten Flächennormalen (24b) erstreckt, und
wobei die erste und zweite Richtung (s₁, s₂) parallel zueinander sind.

9. Röntgendetektor (20; 32) gemäß einem der vorherigen Ansprüche, wobei die Manipulationseinrichtung (72; 72a; 72b; 84; 88; 94a-94i; 96a-96i) ausgebildet ist, einen Winkel (α_{22_24}) zwischen dem ersten und zweiten Detektormodul (32a, 32b) zu verändern.

10. Röntgendetektor (20; 32) gemäß einem der vorherigen Ansprüche, wobei alle Detektormodule (32a-32i) so miteinander gekoppelt sind, dass alle Flächennormalen der jeweiligen Erfassungsebenen in einer Normalenebene liegen.

11. Röntgendetektor gemäß einem der vorherigen Ansprüche, wobei der erste Erfassungsbereich des ersten Detektormoduls (46a) in einer ersten Erfassungsebene liegt und wobei der zweite Erfassungsbereich des zweiten Detektormoduls (46b) in einer zweiten Erfassungsebene liegt,
wobei der Röntgendetektor ein weiteres Detektormodul (48b) mit einem in einer weiteren Erfassungsebene liegenden weiteren Erfassungsbereich umfasst, und
wobei die Manipulationseinrichtung (72) ausgebildet ist, um die erste Erfassungsebene des ersten Detektormoduls (46a), die zweite Erfassungsebene des zweiten Detektormoduls (46b) und die weitere Erfassungsebene des weiteren Detektormoduls (48b) so zueinander auszurichten, dass sich eine erste Flächennormale der ersten Erfassungsebene, eine zweite Flächennormale der zweiten Erfassungsebene und eine weitere Flächennormale der weiteren Erfassungsebene innerhalb eines Referenzbereichs kreuzen,
wobei die erste und/oder zweite Erfassungsebene gegenüber der weiteren Erfassungsebene versetzt ist und der erste und/oder zweite Erfassungsbereich mit dem weiteren Erfassungsbereich überlagert ist.

12. Röntgendetektor (40) gemäß Anspruch 11, wobei die erste und/oder zweite Erfassungsebene tangential auf einer ersten Kreisbahn (42) liegen und die weitere Erfassungsebene tangential auf einer weiteren Kreisbahn (44) liegt, die gegenüber der ersten Kreisbahn (42) versetzt ist, und/oder
wobei mittels Manipulationseinrichtung (72) eine Ausrichtung des ersten und zweiten Detektormoduls (46a, 46b) zueinander so einstellbar ist, dass die erste und zweite Erfassungsebene mit jeweiligen Flächenschwerpunkten tangential auf einer ersten Kreisbahn (42) liegen, und eine Ausrichtung des weiteren Detektormoduls (48b) so einstellbar ist, dass die weitere Erfassungsebene mit einem Flächenschwerpunkt tangential auf einer weiteren Kreisbahn (44) liegt, die mit der ersten Kreisbahn (42) in einer gemeinsamen Normalenebene angeordnet ist.

13. Röntgendetektor (40) gemäß Anspruch 11 oder 12, wobei Mittelpunkte der ersten Kreisbahn (42) und der weiteren Kreisbahn (44), die unterschiedliche, einstellbare Radien aufweisen, in dem Referenzbereich liegen.

14. Röntgendetektor (40) gemäß einem der Ansprüche 1 bis 13, wobei das Drehmoment (M_{b}) ein resultierendes Drehmoment ist, welches über zwei Antriebe an den äußeren Enden eingeleitet wird.

15. Röntgensystem (30) mit folgenden Merkmalen:
einem Röntgendetektor (20; 32; 40) gemäß einem der vorherigen Ansprüche; und
einer Strahlungsquelle (27),
wobei der Radius der Kreisbahn (34) mittels der Manipulationseinrichtung so einstellbar ist, dass ein Brennfleck der Strahlungsquelle (27) dem Mittelpunkt des einstellbaren Radius entspricht.

## Claims

1. An X-ray detector (20; 32) comprising:
a first detector module (32a) having a first detection region (22a) arranged in a first detection plane (22);
a second detector module (32b) having a second detection region (24a) arranged in a second detection plane (24), which is neighboring to the first detector module (32a); and
a first manipulation means (72) configured to orient the first detection plane (22) of the first detector module (32a) and the second detection plane (24) of the second detector module (32b) to each other such that a first normal to surface (22b) of the first detection plane (22) and a second surface to normal (24b) of the second detection plane (24) intersect within a reference region.
a further detector module (32c) having a further detection region arranged in a further detection plane, which is neighboring to the second detector module (32b) and which is arranged relative to the first and second detector modules (32a, 32b) such that the first, second and further detection planes (22, 24) are distributed with their respective centroids tangentially on a circular path (34) at an adjustable radius; and
a further manipulation means (72) configured to orient the further detection plane of the further detector module (32c) relative to the first and second detection planes (22, 24) such that the first and second normals to surface (22b, 24b), together with a further normal to surface of the further detection plane (24), intersect within the reference region,
**characterized in that**
the first and second detector modules (32a, 32b) are connected relative to one another by a first spring (66a) and the second (32b) and the further detector module (32c) are connected relative to one another by a second spring (66b),
wherein
a) the first and second springs (66a; 66b) have an equal spring stiffness and the manipulation means (72a; 72b) are configured to vary a distance (l_{38a_38c}) between the first and further detector modules (32a, 32i) and the springs are configured to orient the X-ray detector (20; 32) along the circular path (34) when varying the distance (l_{38a_38c}), or
wherein
b) the manipulation means (72a, 72b) are configured to provide the first and/or further detector module (32c) with a torque (M_{b}) or a force, and wherein the springs (66a, 66b) are configured to distribute the torque (M_{b}) or the force evenly along all detector modules (32a to 32i) in order to orient the detector modules (32a to 32i) along the circular path (34).

2. An X-ray detector (20; 32) according to claim 1, wherein the manipulation means (72, 72a, 72b) is configured to provide the first and/or further detector module (32a to 32i) with a torque (M_{b}) or a force, and wherein the first, second and further detector module (32a to 32c) are connected by means of springs (66a, 66b) that are configured to distribute the torque (M_{b}) or the force evenly along all detector modules (32a to 32i) in order to orient the detector modules (32a to 32i) along the circular path (34).

3. The X-ray detector (20; 32) in accordance with claim 1 or 2, wherein the first, second and further detector modules (32a to 32i) are coupled to one another such that, when adjusting the radius, an adjustable angle between the first and second normals to surface corresponds to an adjustable angle between the second and further normals to surface.

4. The X-ray detector (20; 32) in accordance with any of the preceding claims, wherein the first detector module (32a) comprises a plurality of sensor elements arranged along a first direction (s₁) and grouped to form a first detector line and the second detector module (32b) comprises a plurality of sensor elements arranged along a second direction (s₂) and grouped to form a second detector line,
wherein the first and second directions (s₁, s₂) are parallel to each other.

5. The X-ray detector (20; 32) in accordance with any of claims 1 to 4, wherein the first detector module (32a) comprises a plurality of sensor modules arranged in a first and a third direction (s₁, t₁) and the second detector module (32b) comprises a plurality of sensor modules arranged in a second and a fourth direction (s₂, t₂),
wherein the first and second directions (s₁, s₂) are parallel to each other and the third and fourth directions (t₁, t₂) extend along the circular path (34) as tangents.

6. The X-ray detector (20; 32) in accordance with any of the preceding claims,
wherein the first detector module (32a) is supported relative to the second detector module (32b) such that the first and second detector modules (32a, 32b) are rotatable relative to each other around a rotational axis (25) for adjusting an orientation of the first and second normals to surface (22b, 24b), and/or
wherein the rotational axis (25) is formed on a common edge (25) of the first and second detector modules (32a, 32b) on the side of the first and second detection regions (22a; 24a); or
wherein the rotational axis (25) is formed between and/or parallel to an edge (25) of the first detector module (32a) on the side of the first detection region (22a) and an edge (25) of the second detector module (32b) on the side of the second detection region (24a).

7. The X-ray detector (20; 32) in accordance with any of the preceding claims,
wherein the first and second detector modules (32a, 32b) are supported relative to each other by a movable connective element (66a; 66b), and
wherein the movable connective element is a spring (66a; 66b) with a defined bending point configured to define a rotational axis (25) around which the first and second detector modules (32a, 32b) are supported relative to each other to be rotatable.

8. The X-ray detector (20; 32) in accordance with any of claims 1 to 7, wherein the first detector module (32a) comprises a scattered radiation grid (54a) along a first direction (s₁) that extends as plate elements perpendicularly from the first detection plane (22a) along the first normal to surface (22b) and the second detector module (32b) comprises a scattered radiation grid (54b) along a second direction (s₂) that extends as plate elements perpendicularly from the second detection plane (24a) along the second normal to surface (24b), and
wherein the first and second directions (s₁, s₂) are parallel to each other.

9. The X-ray detector (20; 32) in accordance with any of the preceding claims,
wherein the manipulation means (72; 72a; 72b; 84; 88; 94a to 94i; 96a to 96i) is configured to vary an angle (α_{22_24}) between the first and second detector modules (32a, 32b).

10. The X-ray detector (20; 32) in accordance with any of the preceding claims,
wherein all the detector modules (32a to 32i) are coupled to one another such that all the normals to surface of the respective detection planes are in a normal plane.

11. The X-ray detector (20; 32) according to any of the preceding claims, wherein the first detection region of the first detection module (46a) is in a first detection plane and wherein the second detection region of the second detector module (46b) is in a second detection plane,
wherein the X-ray detector comprises a further detector module (48b) having a further detection region arranged in a further detection plane; and
wherein the manipulation means (72) is configured to orient the first detection plane of the first detector module (46a), the second detection plane of the second detector module (46b) and the further detection plane of the further detector module (48b) to one another such that a first normal to surface of the first detection plane, a second surface to normal of the second detection plane and a further normal to surface of the further detection region intersect within a reference region,
wherein the first and/or second detection plane(s) is/are offset relative to the further detection plane and the first and/or second detection region(s) is/are overlapped by the further detection region.

12. The X-ray detector (40) in accordance with claim 11, wherein the first and/or second detection plane(s) is/are located tangentially on a first circular path (42) and the further detection plane is located tangentially on a further circular path (44) which is offset relative to the first circular path (42), and/or
wherein an orientation of the first and second detector modules (46a, 46b) to each other may be adjusted by means of manipulation means (72) such that the first and second detection planes are arranged with their respective centroids tangentially on a first circular path (42) and an orientation of the further detector module (48b) may be adjusted such that the further detection plane is arranged with a centroid tangentially on a further circular path (44) which is arranged in a common normal plane with the first circular path (42).

13. The X-ray detector (40) in accordance with claim 11 or 12, wherein centers of the first circular path (42) and the further circular path (44) which comprise different adjustable radii are located in the reference region.

14. The X-ray detector (40) in accordance with any of claims 1 to 13, wherein the torque (M_{b}) is a resulting torque introduced at the outer ends via two drives.

15. An X-ray system (30) comprising:
an X-ray detector (20; 32; 40) in accordance with any of the preceding claims; and
a radiation source (27),
wherein the radius of the circular path (34) may be adjusted by means of the manipulation means such that a focal spot of the radiation source (27) corresponds to the center of the adjustable radius.

## Revendications

1. Détecteur de rayons X (20; 32) aux caractéristiques suivantes:
un premier module de détection (32a) avec une première région de détection (22a) située dans un premier plan de détection (22);
un deuxième module de détection (32b) avec une deuxième région de détection (24a) située dans un deuxième plan de détection (24), lequel est adjacent au premier module de détection (32a); et
un premier moyen de manipulation (72a) qui est conçu pour orienter le premier plan de détection (22) du premier module de détection (32a) et le deuxième plan de détection (24) du deuxième module de détection (32b) l'un par rapport à l'autre de sorte qu'une première normale à la surface (22b) du premier plan de détection (22) et une deuxième normale à la surface (24b) du deuxième plan de détection (24) se croisent dans une zone de référence; et
un autre module de détection (32c) avec une autre zone de détection située dans un autre plan de détection, lequel est adjacent au deuxième module de détection (32b) et est disposé par rapport au premier et au deuxième module de détection (32a, 32b) de sorte que le premier, le deuxième et l'autre plan de détection (22, 24) soient répartis avec des points de gravité de surface respectifs tangentiellement sur une trajectoire circulaire (34) de rayon réglable; et
un autre moyen de manipulation (72b) qui est conçu pour orienter l'autre plan de détection de l'autre module de détection (32c) par rapport au premier et au deuxième plan de détection (22, 24) de sorte que la première et la deuxième normale à la surface (22b, 24b) se croisent ensemble avec une autre normale à la surface de l'autre plan de détection (24) dans la zone de référence,
**caractérisé par le fait que**
le premier et le deuxième module de détection (32a, 32b) sont connectés l'un à l'autre par un premier ressort (66a) et le deuxième (32b) et l'autre module de détection (32c) sont connectés l'un à l'autre par un deuxième ressort (66b),
dans lequel
a) les premier et deuxième ressorts (66a; 66b) présentent la même dureté de ressort et les moyens de manipulation (72a; 72b) sont conçus pour faire varier une distance (l_{38a_38c}) entre le premier et l'autre module de détection (32a, 32i), et les ressorts (66a) sont conçus pour orienter, en cas de variation de la distance (l_{38a_38c}), le détecteur de rayons X (20; 32) le long de la trajectoire circulaire (34),
ou
dans lequel
b) les moyens de manipulation (72a, 72b) sont conçus pour solliciter le premier et/ou l'autre module de détection (32c) avec un couple (M_{b}), et les ressorts (66a, 66b) sont conçus pour répartir le couple (M_{b}) uniformément le long de tous les modules de détection (32a à 32c) pour orienter ainsi les modules de détection (32a à 32c) le long de la trajectoire circulaire (34).

2. Détecteur de rayons X (20; 32) selon la revendication 1, dans lequel le moyen de manipulation (72, 72a, 72b) est conçu pour solliciter le premier et/ou l'autre module de détection (32a à 32i) avec un couple (M_{b}) ou une force, et dans lequel le premier, le deuxième et l'autre module de détection (32a à 32c) sont connectés au moyen de ressorts (66a, 66b) qui sont conçus pour répartir le couple (M_{b}) ou la force uniformément le long de tous les modules de détection (32a à 32i), pour orienter ainsi les modules de détection (32a à 32i) le long de la trajectoire circulaire (34).

3. Détecteur de rayons X (20; 32) selon la revendication 1 ou 2, dans lequel le premier, le deuxième et l'autre module de détection (32a à 32i) sont couplés l'un à l'autre de sorte que, lors d'un réglage du rayon, un angle réglable entre la première et la deuxième normale à la surface corresponde à un angle réglable entre la deuxième et l'autre normale à la surface.

4. Détecteur de rayons X (20; 32) selon l'une des revendications précédentes, dans lequel le premier module de détection (32a) présente une pluralité d'éléments de détection regroupés en une première rangée de détecteurs et disposés dans une première direction (s₁) et le deuxième module de détection (32b) présente une pluralité d'éléments de détection regroupés en une deuxième rangée de détecteurs et disposés dans une deuxième direction (s₂), la première et la deuxième direction (s₁, s₂) étant parallèles entre elles.

5. Détecteur de rayons X (20; 32) selon l'une des revendications 1 à 4, dans lequel le premier module de détection (32a) présente une pluralité de modules de détection disposés dans une première et une troisième direction (s₁, t₁) et le deuxième module de détection (32b) présente une pluralité de modules de détection disposés dans une deuxième et une quatrième direction (s₂, t₂),
dans lequel la première et la deuxième direction (s₁, s₂) sont parallèles entre elles et la troisième et la quatrième direction (t₁, t₂) s'étendent comme tangentes le long de la trajectoire circulaire (34).

6. Détecteur de rayons X (20; 32) selon l'une des revendications précédentes, dans lequel le premier module de détection (32a) est monté par rapport au deuxième module de détection (32b) de sorte que, pour le réglage d'une orientation de la première et de la deuxième normale à la surface (22b, 24b), le premier et le deuxième module de détection (32a, 32b) puissent tourner l'un par rapport à l'autre autour d'un axe de rotation (25), et/ou dans lequel l'axe de rotation (25) est formé sur un bord commun (25) du premier et du deuxième module de détection (32a, 32b) du côté de la première et de la deuxième zone de détection (22a; 24a); ou
dans lequel l'axe de rotation (25) est formé entre et/ou parallèle à un bord (25) du premier module de détection (32a) du côté de la première zone de détection (22a) et un bord (25) du deuxième module de détection (32b) du côté de la deuxième zone de détection (24a).

7. Détecteur de rayons X (20; 32) selon l'une des revendications précédentes, dans lequel le premier et le deuxième module de détection (32a, 32b) sont montés l'un par rapport à l'autre par un élément de connexion mobile (66a, 66b), et
dans lequel l'élément de connexion mobile est un ressort (66a; 66b) avec un coude défini qui est conçu pour définir un axe de rotation (25) autour duquel le premier et le deuxième module de détection (32a, 32b) sont montés de manière à pouvoir tourner l'un par rapport à l'autre.

8. Détecteur de rayons X (20; 32) selon l'une des revendications 1 à 7, dans lequel le premier module de détection (32a) présente une grille de rayonnement diffus (54a) dans une première direction (s₁) qui s'étend comme éléments en forme de plaque perpendiculairement à partir du premier plan de détection (22a) le long de la première normale à la surface (22b), et le deuxième détecteur (32b) présente une grille de rayonnement diffus (54b) dans une deuxième direction (s₂) qui s'étend comme éléments en forme de plaque perpendiculairement à partir du deuxième plan de détection (24a) le long de la deuxième normale à la surface (24b), et
dans lequel la première et la deuxième direction (s₁, s₂) sont parallèles l'une à l'autre.

9. Détecteur de rayons X (20; 32) selon l'une des revendications précédentes, dans lequel le moyen de manipulation (72; 72a; 72b; 84; 88; 94a à 94i; 96a à 96i) est conçu pour modifier un angle (α_{22_24}) entre le premier et le deuxième module de détection (32a, 32b).

10. Détecteur de rayons X (20; 32) selon l'une des revendications précédentes, dans lequel tous les modules de détection (32a à 32i) sont couplés l'un à l'autre de sorte que toutes les normales à la surface des plans de détection respectifs se situent dans un plan normal.

11. Détecteur de rayons X selon l'une des revendications précédentes,
dans lequel la première zone de détection du premier module de détection (46a) se situe dans un premier plan de détection et dans lequel la deuxième zone de détection du deuxième module de détection (46b) se situe dans un deuxième plan de détection,
dans lequel le détecteur de rayons X comporte un autre module de détection (48b) avec une autre zone de détection située dans un autre plan de détection, et
dans lequel le moyen de manipulation (72) est conçu pour orienter le premier plan de détection du premier module de détection (46a), le deuxième plan de détection du deuxième module de détection (46b) et l'autre plan de détection de l'autre module de détection (48b) l'un par rapport à l'autre de sorte qu'une première normale à la surface du premier plan de détection, une deuxième normale à la surface du deuxième plan de détection et une autre normale à la surface de l'autre plan de détection se croisent dans une zone de référence,
dans lequel le premier et/ou le deuxième plan de détection est décalé par rapport à l'autre plan de détection et la première et/ou la deuxième zone de détection se superpose à l'autre zone de détection.

12. Détecteur de rayons X (40) selon la revendication 11, dans lequel le premier et/ou le deuxième plan de détection se situent tangentiellement sur une première trajectoire circulaire (42) et l'autre plan de détection se situe tangentiellement sur une autre trajectoire circulaire (44) qui est décalée par rapport à la première trajectoire circulaire (42), et/ou
dans lequel peut être réglée, à l'aide du moyen de manipulation (72), une orientation du premier et du deuxième module de détection (46a, 46b) l'un par rapport à l'autre de sorte que le premier et le deuxième plan de détection se situent avec des points de gravité de surface respectifs tangentiellement sur une première trajectoire circulaire (42), et une orientation de l'autre module de détection (48b) peut être réglée de sorte que l'autre plan de détection se situe avec un point de gravité de surface tangentiellement sur une autre trajectoire circulaire (44) qui est disposée avec la première trajectoire circulaire (42) dans un plan normal commun.

13. Détecteur de rayons X (40) selon la revendication 11 ou 12, dans lequel les centres de la première trajectoire circulaire (42) et de l'autre trajectoire circulaire (44), qui présentant des rayons réglables différents, se situent dans la zone de référence.

14. Détecteur de rayons X (40) selon l'une des revendications 1 à 13,
dans lequel le couple (M_{b}) est un couple résultant qui est introduit par l'intermédiaire de deux entraînements aux extrémités extérieures.

15. Système à rayons X (30) aux caractéristiques suivantes:
un détecteur de rayons X (20; 32; 40) selon l'une des revendications précédentes; et
une source de rayonnement (27),
dans lequel le rayon de la trajectoire circulaire (34) est réglable à l'aide du moyen de manipulation de sorte qu'un point focal de la source de rayonnement (27) corresponde au centre du rayon réglable.
